# EUROPEAN PATENT APPLICATION

(11) **EP 4 578 459 A1**
(43) Date of publication of application: **02.07.2025**
(21) Application number: 23857367.9
(22) Date of filing: 23.08.2023
(51) Int. Cl.: A61K 39/395, A61K 31/44, A61K 45/00, A61P 35/00, A61P 35/02, A61P 43/00, C07K 16/28

(54) **PHARMACEUTICAL PRODUCT FOR TREATMENT AND/OR PREVENTION OF CANCER**

(30) Priority: 24.08.2022 JP 2022133051
(71) Applicant: Toray Industries, Inc., Tokyo 103-8666 (JP)
(72) Inventor: WASAI Ukei, Kamakura-shi, Kanagawa 248-8555 (JP); OKANO Fumiyoshi, Kamakura-shi, Kanagawa 248-8555 (JP); SAITO Takanori, Kamakura-shi, Kanagawa 248-8555 (JP)
(74) Representative: Mewburn Ellis LLP
(86) International application number: PCT/JP2023/030241
(87) International publication number: WO 2024/043258

(57) **Abstract**

The medicament comprising an antibody or a fragment thereof having an immunological reactivity with CAPRIN-1 protein, and a PDGFR inhibitor together or separately in combination is useful for treating and/or preventing cancer.

## Description

### Technical Field

The present invention relates to a medicament for treatment and/or prevention of a cancer, comprising an antibody against CAPRIN-1 protein, or a fragment thereof, and a PDGFR inhibitor.

### Background Art

Various antibody medicines targeting specific antigen proteins on cancer cells are applied as therapeutic agents for cancers with fewer side effects to cancer treatment because of their cancer specificity. For example, cytoplasmic-activation and proliferation-associated protein 1 (CAPRIN-1) is expressed on cell membrane surfaces of many solid cancers. Antibodies against this CAPRIN-1 protein are known to be promising in pharmaceutical uses for treatment and/or prevention of cancers (Patent Literature 1).

In recent years, treatment methods using combinations of pluralities of therapeutic agents for cancer have been clinically used as standard treatment methods in order to enhance the effectiveness of the therapeutic agents for cancers. In general, for example, breast cancer is treated by a treatment method using a combination of doxorubicin and cyclophosphamide or using a combination of a plurality of anticancer agents such as paclitaxel, trastuzumab, and pertuzumab. Therapeutic agents for cancers comprising anti-CAPRIN-1 antibodies as active ingredients have also been confirmed to have therapeutic effects on the cancers by combinations with chemotherapeutics (Patent Literature 2). However, the treatment of cancer by a combination of chemotherapeutics is not effective for every cancer to which the treatment is applied, and few combinations of chemotherapeutics synergistically drastically enhance therapeutic effects, though some combinations additively enhance therapeutic effects.

In addition, in recent years, development of molecular targeted therapeutics as cancer therapeutics has been vigorously promoted, and one of its targets that has attracted attention is a drug having the action of inhibiting the activity of platelet-derived growth factor receptor (PDGFR). PDGFR is a kind of receptor-type tyrosine kinase, and has two structurally similar subtypes, PDGFRα and PDGFRβ. The structures of both subtypes are characterized by having five extracellular immunoglobulin-like domains, and further having a single transmembrane domain, and an intracellular TK domain (ATP binding pocket and active loop) bisected with a kinase insertion sequence region necessary for binding effector molecules and adaptor molecules. Although these receptor proteins are usually present as monomers on cell membranes, they form dimers as a result of binding of ligands, and the tyrosine residues of PDGFR undergo autophosphorylation to provide a binding site for signaling molecules having a SH2 domain (PLC-γ, Grb2, PI3K, or the like), and downstream signaling pathways such as Ras-MAPK, PI3K-AKT, and PLγ-PKC are activated, thereby promoting cancer proliferation, migration, and the like (Non Patent Literature 1). PDGFR is highly expressed in the stroma of prostate cancers and has been reported to be involved in the progression and prognosis of cancers. However, PDGFR inhibitors have not achieved promising effects in clinical trials, though they have achieved remarkable results in preclinical studies (Non Patent Literature 2).

### Citation List

### Patent Literature

Patent Literature 1: WO2010/016526
Patent Literature 2: WO2011/096535

### Non Patent Literature

Non Patent Literature 1: Genes Dev.2008,22(10):1276-312
Non Patent Literature 2: Mol Aspects Med.2018,62:75-88

### Summary of Invention

### Object to be Achieved

An object of the present invention is to provide a medicament for treatment and/or prevention of a cancer specifically expressing CAPRIN-1 protein on a cell surface.

### Solution to Achieve Object

As a result of intensive studies, the present inventors have found that a combination (combined use) of an antibody against CAPRIN-1 protein, or a fragment thereof, having an immunological reactivity with cancer cells, and a PDGFR inhibitor exerts a very strong antitumor effect. On the basis of these findings, the present invention has been completed.

Specifically, the present invention relates to the following embodiments (1) to (14):
(1) A medicament for treatment and/or prevention of cancer, comprising an antibody or a fragment thereof having an immunological reactivity with CAPRIN-1 protein, and a PDGFR (platelet-derived growth factor receptor) inhibitor together or separately in combination.
(2) The medicament according to (1), wherein the PDGFR inhibitor is Sorafenib.
(3) The medicament according to (1) or (2), wherein the antibody or the fragment thereof has an immunological reactivity with CAPRIN-1 protein having an amino acid sequence shown by any one of the even numbered SEQ ID NOs: 2 to 30, or an amino acid sequence having 80% or more sequence identity with the amino acid sequence.
(4) The medicament according to any of (1) to (3), wherein the antibody or the fragment thereof has an immunological reactivity with an extracellular region of CAPRIN-1 protein present on a cancer cell surface.
(5) The medicament according to any of (1) to (4), wherein the antibody or the fragment thereof has an immunological reactivity with a partial polypeptide of CAPRIN-1 protein, the partial polypeptide having an amino acid sequence shown by any one of SEQ ID NOs: 31 to 35, 296 to 299, 308 and 309, or an amino acid sequence having 80% or more sequence identity with the amino acid sequence.
(6) The medicament according to any of (1) to (5), wherein the antibody is a monoclonal antibody or a polyclonal antibody.
(7) The medicament according to any of (1) to (6), wherein the antibody or the fragment thereof is any one of the following (A) to (M):
   (A) an antibody or a fragment thereof comprising a heavy-chain variable region comprising complementarity determining regions of SEQ ID NOs: 36, 37 and 38 (CDR1, CDR2 and CDR3, respectively) and a light-chain variable region comprising complementarity determining regions of SEQ ID NOs: 40, 41 and 42 (CDR1, CDR2 and CDR3, respectively), and having an immunological reactivity with CAPRIN-1 protein;
   (B) an antibody or a fragment thereof comprising a heavy-chain variable region comprising complementarity determining regions of SEQ ID NOs: 44, 45 and 46 (CDR1, CDR2 and CDR3, respectively) and a light-chain variable region comprising complementarity determining regions of SEQ ID NOs: 48, 49 and 50 (CDR1, CDR2 and CDR3, respectively), and having an immunological reactivity with CAPRIN-1 protein;
   (C) an antibody or a fragment thereof comprising a heavy-chain variable region comprising complementarity determining regions of SEQ ID NOs: 52, 53 and 54 (CDR1, CDR2 and CDR3, respectively) and a light-chain variable region comprising complementarity determining regions of SEQ ID NOs: 56, 57 and 58 (CDR1, CDR2 and CDR3, respectively), and having an immunological reactivity with CAPRIN-1 protein;
   (D) an antibody or a fragment thereof comprising a heavy-chain variable region comprising complementarity determining regions of SEQ ID NOs: 60, 61 and 62 (CDR1, CDR2 and CDR3, respectively) and a light-chain variable region comprising complementarity determining regions of SEQ ID NOs: 64, 65 and 66 (CDR1, CDR2 and CDR3, respectively), and having an immunological reactivity with CAPRIN-1 protein;
   (E) an antibody or a fragment thereof comprising a heavy-chain variable region comprising complementarity determining regions of SEQ ID NOs: 170, 171 and 172 (CDR1, CDR2 and CDR3, respectively) and a light-chain variable region comprising complementarity determining regions of SEQ ID NOs: 173, 174 and 175 (CDR1, CDR2 and CDR3, respectively), and having an immunological reactivity with CAPRIN-1 protein;
   (F) an antibody or a fragment thereof comprising a heavy-chain variable region comprising complementarity determining regions of SEQ ID NOs: 176, 177 and 178 (CDR1, CDR2 and CDR3, respectively) and a light-chain variable region comprising complementarity determining regions of SEQ ID NOs: 179, 180 and 181 (CDR1, CDR2 and CDR3, respectively), and having an immunological reactivity with CAPRIN-1 protein;
   (G) an antibody or a fragment thereof comprising a heavy-chain variable region comprising complementarity determining regions of SEQ ID NOs: 182, 183 and 184 (CDR1, CDR2 and CDR3, respectively) and a light-chain variable region comprising complementarity determining regions of SEQ ID NOs: 185, 186 and 187 (CDR1, CDR2 and CDR3, respectively), and having an immunological reactivity with CAPRIN-1 protein;
   (H) an antibody or a fragment thereof comprising a heavy-chain variable region comprising complementarity determining regions of SEQ ID NOs: 188, 189 and 190 (CDR1, CDR2 and CDR3, respectively) and a light-chain variable region comprising complementarity determining regions of SEQ ID NOs: 191, 192 and 193 (CDR1, CDR2 and CDR3, respectively), and having an immunological reactivity with CAPRIN-1 protein;
   (I) an antibody or a fragment thereof comprising a heavy-chain variable region comprising complementarity determining regions of SEQ ID NOs: 146, 147 and 148 (CDR1, CDR2 and CDR3, respectively) and a light-chain variable region comprising complementarity determining regions of SEQ ID NOs: 149, 150 and 151 (CDR1, CDR2 and CDR3, respectively), and having an immunological reactivity with CAPRIN-1 protein;
   (J) an antibody or a fragment thereof comprising a heavy-chain variable region comprising complementarity determining regions of SEQ ID NOs: 272, 273 and 274 (CDR1, CDR2 and CDR3, respectively) and a light-chain variable region comprising complementarity determining regions of SEQ ID NOs: 275, 276 and 277 (CDR1, CDR2 and CDR3, respectively), and having an immunological reactivity with CAPRIN-1 protein;
   (K) an antibody or a fragment thereof comprising a heavy-chain variable region comprising complementarity determining regions of SEQ ID NOs: 290, 291 and 292 (CDR1, CDR2 and CDR3, respectively) and a light-chain variable region comprising complementarity determining regions of SEQ ID NOs: 293, 294 and 295 (CDR1, CDR2 and CDR3, respectively), and having an immunological reactivity with CAPRIN-1 protein;
   (L) an antibody or a fragment thereof comprising a heavy-chain variable region comprising complementarity determining regions of SEQ ID NOs: 301, 302 and 303 (CDR1, CDR2 and CDR3, respectively) and a light-chain variable region comprising complementarity determining regions of SEQ ID NOs: 305, 306 and 307 (CDR1, CDR2 and CDR3, respectively), and having an immunological reactivity with CAPRIN-1 protein; and
   (M) an antibody or a fragment thereof comprising a heavy-chain variable region comprising complementarity determining regions of SEQ ID NOs: 134, 135 and 136 (CDR1, CDR2 and CDR3, respectively) and a light-chain variable region comprising complementarity determining regions of SEQ ID NOs: 137, 138 and 139 (CDR1, CDR2 and CDR3, respectively), and having an immunological reactivity with CAPRIN-1 protein.
(8) The medicament according to any of (1) to (7), wherein the antibody or the fragment thereof is any one of the following (a) to (al):
   (a) an antibody or a fragment thereof comprising a heavy-chain variable region comprising the amino acid sequence of SEQ ID NO: 39 and a light-chain variable region comprising the amino acid sequence of SEQ ID NO: 43;
   (b) an antibody or a fragment thereof comprising a heavy-chain variable region comprising the amino acid sequence of SEQ ID NO: 47 and a light-chain variable region comprising the amino acid sequence of SEQ ID NO: 51;
   (c) an antibody or a fragment thereof comprising a heavy-chain variable region comprising the amino acid sequence of SEQ ID NO: 55 and a light-chain variable region comprising the amino acid sequence of SEQ ID NO: 59;
   (d) an antibody or a fragment thereof comprising a heavy-chain variable region comprising the amino acid sequence of SEQ ID NO: 63 and a light-chain variable region comprising the amino acid sequence of SEQ ID NO: 67;
   (e) an antibody or a fragment thereof comprising a heavy-chain variable region comprising the amino acid sequence of SEQ ID NO: 68 and a light-chain variable region comprising the amino acid sequence of SEQ ID NO: 69;
   (f) an antibody or a fragment thereof comprising a heavy-chain variable region comprising the amino acid sequence of SEQ ID NO: 70 and a light-chain variable region comprising the amino acid sequence of SEQ ID NO: 71;
   (g) an antibody or a fragment thereof comprising a heavy-chain variable region comprising the amino acid sequence of SEQ ID NO: 72 and a light-chain variable region comprising the amino acid sequence of SEQ ID NO: 73;
   (h) an antibody or a fragment thereof comprising a heavy-chain variable region comprising the amino acid sequence of SEQ ID NO: 74 and a light-chain variable region comprising the amino acid sequence of SEQ ID NO: 75;
   (i) an antibody or a fragment thereof comprising a heavy-chain variable region comprising the amino acid sequence of SEQ ID NO: 76 and a light-chain variable region comprising the amino acid sequence of SEQ ID NO: 77;
   (j) an antibody or a fragment thereof comprising a heavy-chain variable region comprising the amino acid sequence of SEQ ID NO: 78 and a light-chain variable region comprising the amino acid sequence of SEQ ID NO: 79;
   (k) an antibody or a fragment thereof comprising a heavy-chain variable region comprising the amino acid sequence of SEQ ID NO: 80 and a light-chain variable region comprising the amino acid sequence of SEQ ID NO: 81;
   (l) an antibody or a fragment thereof comprising a heavy-chain variable region comprising the amino acid sequence of SEQ ID NO: 82 and a light-chain variable region comprising the amino acid sequence of SEQ ID NO: 83;
   (m) an antibody or a fragment thereof comprising a heavy-chain variable region comprising the amino acid sequence of SEQ ID NO: 84 and a light-chain variable region comprising the amino acid sequence of SEQ ID NO: 85;
   (n) an antibody or a fragment thereof comprising a heavy-chain variable region comprising the amino acid sequence of SEQ ID NO: 86 and a light-chain variable region comprising the amino acid sequence of SEQ ID NO: 87;
   (o) an antibody or a fragment thereof comprising a heavy-chain variable region comprising the amino acid sequence of SEQ ID NO: 88 and a light-chain variable region comprising the amino acid sequence of SEQ ID NO: 89;
   (p) an antibody or a fragment thereof comprising a heavy-chain variable region comprising the amino acid sequence of SEQ ID NO: 90 and a light-chain variable region comprising the amino acid sequence of SEQ ID NO: 91;
   (q) an antibody or a fragment thereof comprising a heavy-chain variable region comprising the amino acid sequence of SEQ ID NO: 92 and a light-chain variable region comprising the amino acid sequence of SEQ ID NO: 93;
   (r) an antibody or a fragment thereof comprising a heavy-chain variable region comprising the amino acid sequence of SEQ ID NO: 94 and a light-chain variable region comprising the amino acid sequence of SEQ ID NO: 95;
   (s) an antibody or a fragment thereof comprising a heavy-chain variable region comprising the amino acid sequence of SEQ ID NO: 96 and a light-chain variable region comprising the amino acid sequence of SEQ ID NO: 97;
   (t) an antibody or a fragment thereof comprising a heavy-chain variable region comprising the amino acid sequence of SEQ ID NO: 98 and a light-chain variable region comprising the amino acid sequence of SEQ ID NO: 99;
   (u) an antibody or a fragment thereof comprising a heavy-chain variable region comprising the amino acid sequence of SEQ ID NO: 100 and a light-chain variable region comprising the amino acid sequence of SEQ ID NO: 101;
   (v) an antibody or a fragment thereof comprising a heavy-chain variable region comprising the amino acid sequence of SEQ ID NO: 102 and a light-chain variable region comprising the amino acid sequence of SEQ ID NO: 103;
   (w) an antibody or a fragment thereof comprising a heavy-chain variable region comprising the amino acid sequence of SEQ ID NO: 104 and a light-chain variable region comprising the amino acid sequence of SEQ ID NO: 105;
   (x) an antibody or a fragment thereof comprising a heavy-chain variable region comprising the amino acid sequence of SEQ ID NO: 106 and a light-chain variable region comprising the amino acid sequence of SEQ ID NO: 107;
   (y) an antibody or a fragment thereof comprising a heavy-chain variable region comprising the amino acid sequence of SEQ ID NO: 108 and a light-chain variable region comprising the amino acid sequence of SEQ ID NO: 109;
   (z) an antibody or a fragment thereof comprising a heavy-chain variable region comprising the amino acid sequence of SEQ ID NO: 110 and a light-chain variable region comprising the amino acid sequence of SEQ ID NO: 111;
   (aa) an antibody or a fragment thereof comprising a heavy-chain variable region comprising the amino acid sequence of SEQ ID NO: 112 and a light-chain variable region comprising the amino acid sequence of SEQ ID NO: 113;
   (ab) an antibody or a fragment thereof comprising a heavy-chain variable region comprising the amino acid sequence of SEQ ID NO: 114 and a light-chain variable region comprising the amino acid sequence of SEQ ID NO: 115;
   (ac) an antibody or a fragment thereof comprising a heavy-chain variable region comprising the amino acid sequence of SEQ ID NO: 116 and a light-chain variable region comprising the amino acid sequence of SEQ ID NO: 117;
   (ad) an antibody or a fragment thereof comprising a heavy-chain variable region comprising the amino acid sequence of SEQ ID NO: 118 and a light-chain variable region comprising the amino acid sequence of SEQ ID NO: 119;
   (ae) an antibody or a fragment thereof comprising a heavy-chain variable region comprising the amino acid sequence of SEQ ID NO: 120 and a light-chain variable region comprising the amino acid sequence of SEQ ID NO: 121;
   (af) an antibody or a fragment thereof comprising a heavy-chain variable region comprising the amino acid sequence of SEQ ID NO: 122 and a light-chain variable region comprising the amino acid sequence of SEQ ID NO: 123;
   (ag) an antibody or a fragment thereof comprising a heavy-chain variable region comprising the amino acid sequence of SEQ ID NO: 124 and a light-chain variable region comprising the amino acid sequence of SEQ ID NO: 125;
   (ah) an antibody or a fragment thereof comprising a heavy-chain variable region comprising the amino acid sequence of SEQ ID NO: 126 and a light-chain variable region comprising the amino acid sequence of SEQ ID NO: 127;
   (ai) an antibody or a fragment thereof comprising a heavy-chain variable region comprising the amino acid sequence of SEQ ID NO: 128 and a light-chain variable region comprising the amino acid sequence of SEQ ID NO: 129;
   (aj) an antibody or a fragment thereof comprising a heavy-chain variable region comprising the amino acid sequence of SEQ ID NO: 130 and a light-chain variable region comprising the amino acid sequence of SEQ ID NO: 131;
   (ak) an antibody or a fragment thereof comprising a heavy-chain variable region comprising the amino acid sequence of SEQ ID NO: 132 and a light-chain variable region comprising the amino acid sequence of SEQ ID NO: 133; and
   (al) an antibody or a fragment thereof comprising a heavy-chain variable region comprising the amino acid sequence of SEQ ID NO: 300 and a light-chain variable region comprising the amino acid sequence of SEQ ID NO: 304.
(9) The medicament according to any of (1) to (8), wherein the antibody is a human antibody, a humanized antibody, a chimeric antibody or a single chain antibody.
(10) The medicament according to any of (1) to (9), wherein the cancer is a cancer expressing CAPRIN-1 protein on a cell membrane surface.
(11) The medicament according to any of (1) to (10), wherein the cancer is colon cancer, lung cancer, prostate cancer, ovarian cancer, pancreatic cancer, kidney cancer, breast cancer, gastric cancer, bile duct cancer, thyroid cancer, melanoma, renal cell cancer, Hodgkin's lymphoma, head and neck cancer, mesothelioma, colorectal cancer, esophageal cancer, gastroesophageal junction cancer, hepatocellular cancer, glioblastoma, urothelial cancer, urinary bladder cancer, uterus cancer, primary central nervous system lymphoma, primary testicular lymphoma, biliary tract cancer, brain tumor, leukemia, liver cancer, sarcoma, fibrosarcoma, mastocytoma, adrenal cortex cancer, Ewing's tumor, testicle cancer, basal cell cancer, lymphoma, multiple myeloma, Paget's disease or skin cancer.
(12) An agent increasing drug efficacy of a pharmaceutical composition for treatment and/or prevention of cancer comprising an antibody or a fragment thereof having an immunological reactivity with CAPRIN-1 protein as an active ingredient, wherein the agent comprises a PDGFR inhibitor as an active ingredient.
(13) An agent increasing drug efficacy of a pharmaceutical composition for treatment and/or prevention of cancer comprising a PDGFR inhibitor as an active ingredient, wherein the agent comprises an antibody or a fragment thereof having an immunological reactivity with CAPRIN-1 protein as an active ingredient.
(14) A method for treating and/or preventing cancer, comprising administering an antibody or a fragment thereof having an immunological reactivity with CAPRIN-1 protein, and a PDGFR inhibitor together or separately to a subject.

### Advantageous Effects of Invention

The combination of an antibody against CAPRIN-1 protein or a fragment thereof and a PDGFR inhibitor according to the present invention exerts a stronger antitumor effect than that of the antibody against CAPRIN-1 protein alone or an existing chemotherapeutic alone. In particular, although the signaling system targeted by the PDGFR inhibitor is not directly related to the immune system, the present invention has the effect of increasing the phagocytic activity against cancer cells by immunocytes. Thus, the combination of the antibody or a fragment thereof against CAPRIN-1 protein and the PDGFR inhibitor is effective for the treatment or prevention of cancers.

### Brief Description of Drawings

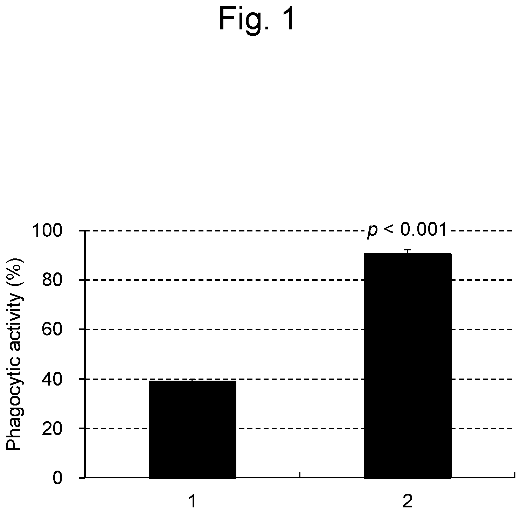

[Figure 1] Figure 1 is a diagram showing human monocytic cell (THP-1)-mediated phagocytic activity of a combination of an anti-CAPRIN-1 antibody and sorafenib against a human colon cancer cell line (HCT116). Reference number 1: a test group without a drug used in combination. Reference number 2; sorafenib combination test group (10 µM). The graph shows an average value of data obtained at N = 3 per sample. Error bars depict standard deviation (S.D.). As a result of conducting Student's t test, the phagocytic activity of the drug combination test group was significantly higher than that of the test group without a drug used in combination (p < 0.001; significance level: 5%).

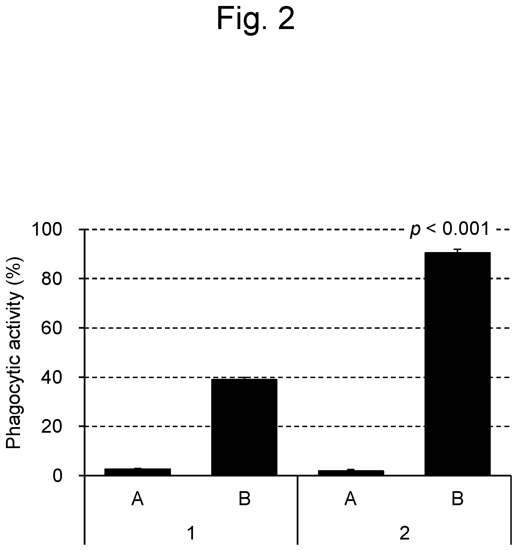

[Figure 2] Figure 2 is a diagram showing human monocytic cell (THP-1)-mediated phagocytic activity of a combination of an anti-CAPRIN-1 antibody and sorafenib against a human colon cancer cell line (HCT116). Reference number 1: a test group without a drug used in combination. Reference number 2; sorafenib combination test group (10 µM). Reference number A; control IgG antibody treatment group; Reference number B; anti-CAPRIN-1 antibody treatment group. The graph shows an average value of data obtained at N = 3 per sample. Error bars depict standard deviation (S.D.). As a result of conducting Dunnett's test, the phagocytic activity of the drug combination test group was significantly higher than that of the test group without a drug used in combination, in the anti-CAPRIN-1 antibody treatment group (p < 0.001; significance level: 5%).

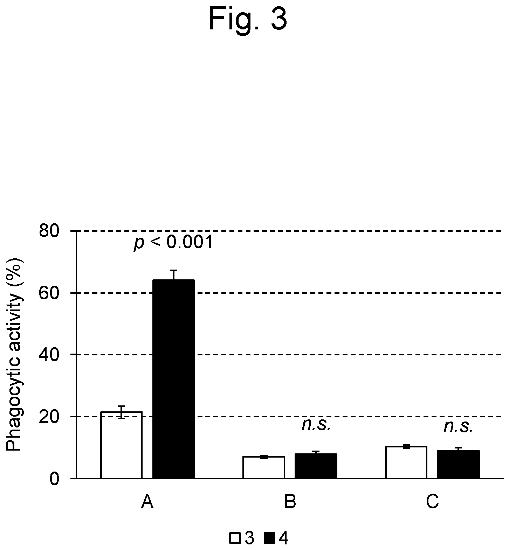

[Figure 3] Figure 3 is a diagram showing human monocytic cell (THP-1)-mediated phagocytic activity of anti-CAPRIN-1 antibody, anti-CD20 antibody or anti-HER-2 antibody each alone, or a combination of each of these antibodies and sorafenib against a human colon cancer cell line (HCT116). Reference number 3: a test group without a drug used in combination. Reference number 4; sorafenib combination test group (10 µM). A; anti-CAPRIN-1 antibody test group (1 µM), B; anti-CD20 antibody test group (1 µM), C; anti-HER-2 antibody test group (1 µM). The graph shows an average value of data obtained at N = 3 per sample. Error bars depict standard deviation (S.D.). As a result of conducting Dunnett's test, the phagocytic activity of the drug combination test group was significantly higher than that of the test group without a drug used in combination, in the anti-CAPRIN-1 antibody test group (p < 0.001, significance level: 5%). In the anti-CD20 antibody or anti-HER-2 antibody test group, there was no significant change in the phagocytic activity of the drug combination test group compared to the test group without a drug used in combination. n.s.; Not Significant.

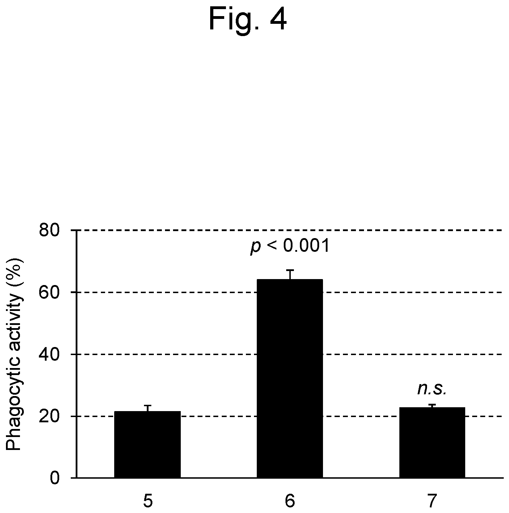

[Figure 4] Figure 4 is a diagram showing human monocytic cell (THP-1)-mediated phagocytic activity of an anti-CAPRIN-1 antibody alone or a combination of the anti-CAPRIN-1 antibody and sorafenib or cisplatin against a human colon cancer cell line (HCT116). Reference number 5: a test group without a drug used in combination. Reference number 6; sorafenib combination test group (10 µM), Reference number 7; cisplatin combination test group (1 µM). The graph shows an average value of data obtained at N = 3 per sample. Error bars depict standard deviation (S.D.). Statistical test; Dunnett's test, p < 0.001, significance level 5%. n.s.; Not Significant.

### Description of Embodiments

The antitumor effect of the combination of an antibody against CAPRIN-1 protein or a fragment thereof (hereinafter, referred to as an "anti-CAPRIN-1 antibody") and a PDGFR inhibitor, used in the present invention is preferably evaluated by examining *in vitro* the phagocytic activity against cancer cells by immunocytes in coculturing the cancer cells and the immunocytes as mentioned later. The immunocyte used in evaluating the antitumor effect *in vitro* may be any cell as long as the immunocyte is a blood cell having phagocytic activity, and preferably, is a human monocytic cell (THP-1 or U937). When an antibody binds to a cancer cell, this is recognized by an immunocyte so that the cancer cell is killed via phagocytic activity by the immunocytes. Therefore, an *in vivo* antitumor effect can be predicted by evaluating the *in vitro* antitumor effect.

The term "combination" (or "combined use") described herein refers to simultaneous administration or addition, or administration or addition in a predetermined interval of the anti-CAPRIN-1 antibody and the PDGFR inhibitor as independent active ingredients to the same organism or cell. The interval may be simultaneous administration or may be 30 minutes later, 1 hour later, 3 hours later, 6 hours later, 12 hours later, 1 day later, 2 days later, 3 days later, 5 days later, 7 days later, 2 weeks later, 3 weeks later, or 4 weeks later. During one of the anti-CAPRIN-1 antibody or the PDGFR inhibitor exhibits its antitumor effect, the other can be administered or added.

The term "comprising together or separately in combination" described herein refers to comprising a plurality of drugs in a form that allows the drugs to be administered simultaneously or separately to a patient. The form may be, for example, the form of a so-called mixed formulation in which a plurality of drugs are mixed, or may be the form of a so-called kit formulation (pharmaceutical kit) comprising a plurality of drugs as individual formulations. The form also encompasses the form of a kit formulation comprising a plurality of drugs in any combination in two or more of formulations.

Such a kit formulation according to the present invention may be, for example, a kit formulation comprising: a formulation (or a pharmaceutical composition) comprising the anti-CAPRIN-1 antibody and a formulation (or a pharmaceutical composition) comprising the PDGFR inhibitor.

The anti-CAPRIN-1 antibody according to the present invention may be a monoclonal antibody or a polyclonal antibody and is preferably a monoclonal antibody. The antibody of the present invention may be any type of antibody as long as it can exhibit antitumor effect. The antibody is a recombinant antibody, a human antibody, a humanized antibody, a chimeric antibody, or a non-human animal antibody.

Subjects being subjected to treatment and/or prevention of cancer according to the present invention are mammals such as primates, pet animals, livestock animals, or sport animals and preferably dogs and cats, and more preferably humans.

Medicaments comprising an anti-CAPRIN-1 antibody and a PDGFR inhibitor as active ingredients, and methods for treating and/or preventing cancers, related to the present invention, will be explained below.

### <Anti-CAPRIN-1 antibody>

Among CAPRIN-1 proteins having an amino acid sequence shown by any one of the even numbered SEQ ID NOs: 2 to 30, which are specific examples of antigens having an immunological reactivity with the anti-CAPRIN-1 antibody used in the present invention, the amino acid sequences shown by SEQ ID NOs: 6, 8, 10, 12 and 14 are amino acid sequences of canine CAPRIN-1 proteins; the amino acid sequences shown by SEQ ID NOs: 2 and 4 are amino acid sequences of human CAPRIN-1 proteins; the amino acid sequence shown by SEQ ID NO: 16 is an amino acid sequence of a bovine CAPRIN-1 protein; the amino acid sequence shown by SEQ ID NO: 18 is an amino acid sequence of a horse CAPRIN-1 protein; the amino acid sequences shown by SEQ ID NOs: 20, 22, 24, 26 and 28 are amino acid sequences of mouse CAPRIN-1 proteins; and the amino acid sequence shown by SEQ ID NO: 30 is an amino acid sequence of a chicken CAPRIN-1 protein.

The anti-CAPRIN-1 antibody used in the present invention may have an immunological reactivity with a CAPRIN-1 protein variant having 80% or more, preferably 90% or more, more preferably 95% or more, and further preferably 99% or more sequence identity to the amino acid sequence shown by any one of the even numbered SEQ ID NOs: 2 to 30. The term "% sequence identity" as used herein means a percentage (%) of the number of identical amino acids (or nucleotides) to the total number of amino acids (or nucleotides) in the case that two sequences are aligned such that maximum similarity can be achieved with or without introduction of gaps.

In the present invention, the anti-CAPRIN-1 antibody refers to an antibody or a fragment (antigen binding fragment) thereof having an immunological reactivity with a full-length CAPRIN-1 protein or a fragment thereof. The term "immunological reactivity" used herein indicates the characteristics of an antibody specifically binding *in vivo* to CAPRIN-1 protein or a partial polypeptide thereof.

The anti-CAPRIN-1 antibody used in the present invention may be a monoclonal antibody or a polyclonal antibody.

Polyclonal antibodies having an immunological reactivity with a full-length CAPRIN-1 protein or a fragment thereof (anti-CAPRIN-1 polyclonal antibodies) can be obtained, for example, in a manner described below. Serum is obtained after mice, human antibody-producing mice, rats, rabbits, chickens, or the like are immunized using a naturally occurring CAPRIN-1 protein or the protein fused with GST or the like, or a partial peptide thereof. The obtained serum is purified via ammonium sulfate precipitation, protein A, protein G, DEAE ion-exchange columns, affinity columns to which a CAPRIN-1 protein or a partial peptide thereof is coupled, or the like.

Nucleotide sequences and amino acid sequences of CAPRIN-1 and homologs thereof used in the immunization can be obtained by, for example, accessing the website of GenBank (NCBI, USA) and using the BLAST or FASTA algorithm (Karlin and Altschul, Proc. Natl. Acad. Sci. USA, 90: 5873-5877, 1993; and Altschul et al., Nucleic Acids Res. 25: 3389-3402, 1997). Methods for producing CAPRIN-1 protein can be obtained with reference to WO2014/012479 or may employ cells or the like expressing CAPRIN-1 protein.

Monoclonal antibodies having an immunological reactivity with a full-length CAPRIN-1 protein or a fragment thereof (anti-CAPRIN-1 monoclonal antibodies) can be obtained, for example, in a manner described below. Breast cancer cells SK-BR-3 expressing CAPRIN-1, a full-length CAPRIN-1 protein or a fragment thereof, or the like is administered to mice for immunization. Splenocytes separated from the mice are fused with myeloma cells. Clones capable of producing anti-CAPRIN-1 monoclonal antibodies can be selected from the obtained fusion cells (hybridomas) to obtain these antibodies. The antibodies produced from the selected hybridomas can be obtained in the same way as the aforementioned method for purifying polyclonal antibodies.

The antibody used in the present invention includes human antibodies, humanized antibodies, chimeric antibodies, non-human animal antibodies and single chain antibodies.

For human antibodies, human lymphocytes infected with EB virus are sensitized with a protein, protein-expressing cells, or a lysate thereof. The sensitized lymphocytes are fused with human-derived myeloma cells such as U266 cells. Antibodies having an immunological reactivity with a full-length CAPRIN-1 protein or a fragment thereof can be obtained from the obtained fusion cells.

A humanized antibody is a modified antibody, and it is sometimes referred to as a reshaped human antibody. It is known that a humanized antibody is constructed by transplanting complementarity determining regions of an immunized animal-derived antibody into complementarity determining regions of a human antibody. In addition, a general gene recombinant technique therefor is well known. Specifically, a DNA sequence designed in a manner that allows complementarity determining regions of mouse or rabbit antibody to be ligated to human antibody framework regions is synthesized by the PCR method using several oligonucleotides prepared in such a manner that the oligonucleotides have portions overlapping each other at an end of each thereof. A humanized antibody can be obtained by ligating the above obtained DNA to DNA encoding a human antibody constant region, incorporating the resultant into an expression vector, and introducing the vector into a host for antibody production (see EP-A-239400 and WO96/02576). Framework regions of human antibody ligated to each other via complementarity determining regions are selected on the assumption that complementarity determining regions can form a good antigen binding site. If necessary, amino acids in framework regions of an antibody variable region may be substituted in such a manner that complementarity determining regions in a reshaped human antibody form an appropriate antigen binding site (Sato K. et al., Cancer Research 1993, 53:851-856). In addition, the framework regions may be substituted with framework regions from a different human antibody (see WO99/51743).

In general, antibodies are heteromultimeric glycoproteins each comprising at least two heavy chains and two light chains. Antibodies each comprise two identical light chains and two identical heavy chains. Each heavy chain has a heavy-chain variable region at one end thereof, to which some constant regions are bound in series. Each light chain has a light-chain variable region at one end thereof to which some constant regions are bound in series. Variable regions have specific variable regions, which are called complementarity determining regions (CDRs) and impart binding specificity to an antibody. A relatively conserved portion in a variable region is called a framework region (FR). A complete heavy-chain or light-chain variable region comprises 4 FRs connected to each other via 3 CDRs (CDR1 to CDR3).

Sequences of human-derived heavy-chain and light-chain constant regions and variable regions can be obtained from, for example, NCBI (USA; GenBank, UniGene, etc.). For example, for a human IgG1 heavy-chain constant region, see registration No. J00228; for a human IgG2 heavy-chain constant region, see registration No. J00230; for a human light chain κ constant region, see sequences such as registration Nos. V00557, X64135, and X64133; and for a human light chain λ constant region, see sequences such as registration Nos. X64132 and X64134.

A chimeric antibody is an antibody produced by combining sequences from different animals. An example thereof is an antibody consisting of mouse antibody heavy-chain and light-chain variable regions and human antibody heavy-chain and light-chain constant regions. Such a chimeric antibody can be produced by a known method. For example, it can be obtained by ligating DNA encoding an antibody V region to DNA encoding a human antibody C region, incorporating the resultant into an expression vector, and introducing the vector into a host for antibody production.

Non-human animal antibodies are obtained by immunizing non-human animals with sensitizing antigens according to a known method or by intraperitoneally, intracutaneously, or subcutaneously injecting sensitizing antigens into animals such as mice as a general method. For injecting sensitizing antigens, an appropriate amount of various adjuvants including CFA (Freund's complete adjuvant) is mixed therewith and the mixture is administered to animals several times. After immunization of animals and confirmation of an anti-CAPRIN-1 antibody contained in serum, the serum is obtained and the non-human animal antibody can be obtained by purification via ammonium sulfate precipitation, protein A, protein G, DEAE ion-exchange columns, affinity columns to which a CAPRIN-1 protein or a partial peptide thereof is coupled, or the like, as mentioned above. In the case of obtaining monoclonal antibodies from non-human animals, a monoclonal antibody is obtained by collecting immunocytes from the immunized animals and subjecting them to cell fusion with myeloma cells. The cell fusion of immunocytes with myeloma cells can be carried out according to a known method (see Kohler, G. and Milstein, C. Methods Enzymol. (1981) 73, 3-46).

The antibody used in the present invention can also be obtained as a gene recombinant antibody produced by cloning an antibody gene from a hybridoma, incorporating the clone into an adequate vector, introducing the vector into a host, and producing the antibody by using a gene recombinant technique (see Carl, A.K. Borrebaeck, James, W. Larrick, THERAPEUTIC MONOCLONAL ANTIBODIES, Published in the United Kingdom by MACMILLAN PUBLISHERS LTD, 1990).

Amino acids in a variable region (e.g., FR) or a constant region in the anti-CAPRIN-1 antibody used in the present invention may be substituted with different amino acids. The amino acid substitution is a substitution of 1 or more, for example, less than 15, less than 10, not more than 8, not more than 6, not more than 5, not more than 4, not more than 3, or not more than 2 amino acids, preferably 1 to 9 amino acids. A substituted antibody should have characteristics of specifically binding to the antigen and binding affinity for the antigen equivalent to or more than those of an unsubstituted antibody and should be an antibody that causes no rejection when applied to humans. The amino acid substitution is preferably a conservative amino acid substitution, which is a substitution between amino acids having similar characteristics in terms of charge, side chains, polarity, aromaticity, and the like. For example, characteristically similar amino acids can be classified into the following types: basic amino acids (arginine, lysine, and histidine); acidic amino acids (aspartic acid and glutamic acid); uncharged polar amino acids (glycine, asparagine, glutamine, serine, threonine, cysteine, and tyrosine); nonpolar amino acids (leucine, isoleucine, alanine, valine, proline, phenylalanine, tryptophan, and methionine); branched-chain amino acids (threonine, valine, isoleucine); and aromatic amino acids (phenylalanine, tyrosine, tryptophan, and histidine).

The anti-CAPRIN-1 antibody used in the present invention is expected to have a stronger antitumor effect when having higher binding affinity for CAPRIN-1 protein on cancer cell surfaces. Association constant (affinity constant) Ka (kon/koff) is preferably at least 10⁷ M⁻¹, at least 10⁸ M⁻¹, at least 5 × 10⁸ M⁻¹, at least 10⁹ M⁻¹, at least 5 × 10⁹ M⁻¹, at least 10¹⁰ M⁻¹, at least 5 × 10¹⁰ M⁻¹, at least 10¹¹ M⁻¹, at least 5 × 10¹¹ M⁻¹, at least 10¹² M⁻¹, or at least 10¹³ M⁻¹.

The anti-CAPRIN-1 antibody used in the present invention may be chemically modified. Examples of such an antibody modifier can include antibodies bound to various molecules such as polyethylene glycol (PEG) and antitumor compounds (for example, antitumor agents listed below). Regarding antibody modifiers of the present invention, substances that bind to an antibody are not limited. Such an antibody modifier can be obtained by chemically modifying an obtained antibody. Methods of such modification have been already established in the field related to the present invention.

The binding strength of the anti-CAPRIN-1 antibody used in the present invention against effector cells can be improved by substituting 1, 2 or several amino acids in the heavy-chain constant region of the antibody or by removing fucose bound to N-acetylglucosamine in a N-glycoside-linked sugar chain bound to the heavy-chain constant region. The anti-CAPRIN-1 antibody described above may have the amino acid substitution alone or may be a composition with an antibody bound to fucose.

Antibodies in which 1, 2 or several amino acids in the heavy-chain constant region have been substituted can be produced with reference to, for example, WO2004/063351, WO2011/120135, U.S. Patent No. 8388955, WO2011/005481, U.S. Patent No. 6737056, and WO2005/063351.

Antibodies in which fucose bound to N-acetylglucosamine in a N-glycoside-linked sugar chain in the heavy-chain constant region has been removed, or producing cells thereof can be produced with reference to U.S. Patent No. 6602684, EP Patent No. 1914244, and U.S. Patent No. 7579170. Compositions of antibodies in which fucose bound to N-acetylglucosamine in a N-glycoside-linked sugar chain bound to the heavy-chain constant region has been removed, with antibodies bound to fucose, or producing cells thereof can be produced with reference to, for example, U.S. Patent No. 8642292.

The anti-CAPRIN-1 polyclonal antibody and the anti-CAPRIN-1 monoclonal antibody used in the present invention, methods for producing or purifying antibodies and methods for producing a CAPRIN-1 protein or partial polypeptide thereof used in immunization can be obtained with reference to WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125630, WO2013/125640, WO2013/147169, WO2013/147176 and WO2015/020212.

Specific examples of the anti-CAPRIN-1 antibody according to the present invention include anti-CAPRIN-1 antibodies described in WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125630, WO2013/125640, WO2013/147169, WO2013/147176 and WO2015/020212 mentioned above. Preferred examples of the anti-CAPRIN-1 antibody include the following.

An antibody or a fragment thereof having an immunological reactivity with a partial polypeptide of CAPRIN-1 protein having the amino acid sequence shown by SEQ ID NO: 2 or SEQ ID NO: 4 or an amino acid sequence having 80% or more (preferably 85% or more, more preferably 90% or more, further preferably 95% or more, and still further preferably 99% or more) sequence identity to the amino acid sequence.

An antibody or a fragment thereof having an immunological reactivity with a partial polypeptide of CAPRIN-1 protein, the partial polypeptide having the amino acid sequence shown by SEQ ID NO: 31 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 36, 37 and 38 (CDR1, CDR2 and CDR3, respectively) and a light-chain variable region comprising complementarity determining regions of SEQ ID NOs: 40, 41 and 42 (CDR1, CDR2 and CDR3, respectively), and having an immunological reactivity with CAPRIN-1 protein, an antibody or a fragment thereof comprising a heavy-chain variable region comprising complementarity determining regions of SEQ ID NOs: 140, 141 and 142 (CDR1, CDR2 and CDR3, respectively) and a light-chain variable region comprising complementarity determining regions of SEQ ID NOs: 143, 144 and 145 (CDR1, CDR2 and CDR3, respectively), and having an immunological reactivity with CAPRIN-1 protein, or an antibody or a fragment thereof comprising a heavy-chain variable region comprising complementarity determining regions of SEQ ID NOs: 164, 165 and 166 (CDR1, CDR2 and CDR3, respectively) and a light-chain variable region comprising complementarity determining regions of SEQ ID NOs: 167, 168 and 169 (CDR1, CDR2 and CDR3, respectively), and having an immunological reactivity with CAPRIN-1 protein, and more preferably an antibody or a fragment thereof comprising a heavy-chain variable region comprising the amino acid sequence of SEQ ID NO: 39 and a light-chain variable region comprising the amino acid sequence of SEQ ID NO: 43, an antibody or a fragment thereof comprising a heavy-chain variable region comprising the amino acid sequence of SEQ ID NO: 70 and a light-chain variable region comprising the amino acid sequence of SEQ ID NO: 71, or an antibody or a fragment thereof comprising a heavy-chain variable region comprising the amino acid sequence of SEQ ID NO: 78 and a light-chain variable region comprising the amino acid sequence of SEQ ID NO: 79.

An antibody or a fragment thereof having an immunological reactivity with a partial polypeptide of CAPRIN-1 protein, the partial polypeptide having the amino acid sequence shown by SEQ ID NO: 33 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 60, 61 and 62 (CDR1, CDR2 and CDR3, respectively) and a light-chain variable region comprising complementarity determining regions of SEQ ID NOs: 64, 65 and 66 (CDR1, CDR2 and CDR3, respectively), and having an immunological reactivity with CAPRIN-1 protein, and more preferably an antibody or a fragment thereof comprising a heavy-chain variable region comprising the amino acid sequence of SEQ ID NO: 63 and a light-chain variable region comprising the amino acid sequence of SEQ ID NO: 67.

An antibody or a fragment thereof having an immunological reactivity with a partial polypeptide of CAPRIN-1 protein, the partial polypeptide having the amino acid sequence shown by SEQ ID NO: 32 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 52, 53 and 54 (CDR1, CDR2 and CDR3, respectively) and a light-chain variable region comprising complementarity determining regions of SEQ ID NOs: 56, 57 and 58 (CDR1, CDR2 and CDR3, respectively), and having an immunological reactivity with CAPRIN-1 protein, and more preferably an antibody or a fragment thereof comprising a heavy-chain variable region comprising the amino acid sequence of SEQ ID NO: 55 and a light-chain variable region comprising the amino acid sequence of SEQ ID NO: 59.

An antibody or a fragment thereof having an immunological reactivity with a partial polypeptide of CAPRIN-1 protein, the partial polypeptide having the amino acid sequence shown by SEQ ID NO: 34 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 170, 171 and 172 (CDR1, CDR2 and CDR3, respectively) and a light-chain variable region comprising complementarity determining regions of SEQ ID NOs: 173, 174 and 175 (CDR1, CDR2 and CDR3, respectively), and having an immunological reactivity with CAPRIN-1 protein, or an antibody or a fragment thereof comprising a heavy-chain variable region comprising complementarity determining regions of SEQ ID NOs: 176, 177 and 178 (CDR1, CDR2 and CDR3, respectively) and a light-chain variable region comprising complementarity determining regions of SEQ ID NOs: 179, 180 and 181 (CDR1, CDR2 and CDR3, respectively), and having an immunological reactivity with CAPRIN-1 protein, and more preferably an antibody or a fragment thereof comprising a heavy-chain variable region comprising the amino acid sequence of SEQ ID NO: 80 and a light-chain variable region comprising the amino acid sequence of SEQ ID NO: 81, or an antibody or a fragment thereof comprising a heavy-chain variable region comprising the amino acid sequence of SEQ ID NO: 82 and a light-chain variable region comprising the amino acid sequence of SEQ ID NO: 83.

An antibody or a fragment thereof having an immunological reactivity with a partial polypeptide of CAPRIN-1 protein, the partial polypeptide having the amino acid sequence shown by SEQ ID NO: 35 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 182, 183 and 184 (CDR1, CDR2 and CDR3, respectively) and a light-chain variable region comprising complementarity determining regions of SEQ ID NOs: 185, 186 and 187 (CDR1, CDR2 and CDR3, respectively), and having an immunological reactivity with CAPRIN-1 protein, or an antibody or a fragment thereof comprising a heavy-chain variable region comprising complementarity determining regions of SEQ ID NOs: 188, 189 and 190 (CDR1, CDR2 and CDR3, respectively) and a light-chain variable region comprising complementarity determining regions of SEQ ID NOs: 191, 192 and 193 (CDR1, CDR2 and CDR3, respectively), and having an immunological reactivity with CAPRIN-1 protein, and more preferably an antibody or a fragment thereof comprising a heavy-chain variable region comprising the amino acid sequence of SEQ ID NO: 84 and a light-chain variable region comprising the amino acid sequence of SEQ ID NO: 85, or an antibody or a fragment thereof comprising a heavy-chain variable region comprising the amino acid sequence of SEQ ID NO: 86 and a light-chain variable region comprising the amino acid sequence of SEQ ID NO: 87.

An antibody or a fragment thereof comprising a heavy-chain variable region comprising complementarity determining regions of SEQ ID NOs: 44, 45 and 46 (CDR1, CDR2 and CDR3, respectively) and a light-chain variable region comprising complementarity determining regions of SEQ ID NOs: 48, 49 and 50 (CDR1, CDR2 and CDR3, respectively), and having an immunological reactivity with CAPRIN-1 protein, and preferably an antibody or a fragment thereof comprising a heavy-chain variable region comprising the amino acid sequence of SEQ ID NO: 47 and a light-chain variable region comprising the amino acid sequence of SEQ ID NO: 51.

An antibody or a fragment thereof having an immunological reactivity with a partial polypeptide of CAPRIN-1 protein, the partial polypeptide having the amino acid sequence shown by SEQ ID NO: 296 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 146, 147 and 148 (CDR1, CDR2 and CDR3, respectively) and a light-chain variable region comprising complementarity determining regions of SEQ ID NOs: 149, 150 and 151 (CDR1, CDR2 and CDR3, respectively), and having an immunological reactivity with CAPRIN-1 protein, and more preferably an antibody or a fragment thereof comprising a heavy-chain variable region comprising the amino acid sequence of SEQ ID NO: 72 and a light-chain variable region comprising the amino acid sequence of SEQ ID NO: 73.

An antibody or a fragment thereof having an immunological reactivity with a partial polypeptide of CAPRIN-1 protein, the partial polypeptide having the amino acid sequence shown by SEQ ID NO: 297 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 272, 273 and 274 (CDR1, CDR2 and CDR3, respectively) and a light-chain variable region comprising complementarity determining regions of SEQ ID NOs: 275, 276 and 277 (CDR1, CDR2 and CDR3, respectively), and having an immunological reactivity with CAPRIN-1 protein, and more preferably an antibody or a fragment thereof comprising a heavy-chain variable region comprising the amino acid sequence of SEQ ID NO: 114 and a light-chain variable region comprising the amino acid sequence of SEQ ID NO: 115.

An antibody or a fragment thereof having an immunological reactivity with a partial polypeptide of CAPRIN-1 protein, the partial polypeptide having the amino acid sequence shown by SEQ ID NO: 298 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 290, 291 and 292 (CDR1, CDR2 and CDR3, respectively) and a light-chain variable region comprising complementarity determining regions of SEQ ID NOs: 293, 294 and 295 (CDR1, CDR2 and CDR3, respectively), and having an immunological reactivity with CAPRIN-1 protein, and more preferably an antibody or a fragment thereof comprising a heavy-chain variable region comprising the amino acid sequence of SEQ ID NO: 120 and a light-chain variable region comprising the amino acid sequence of SEQ ID NO: 121.

An antibody or a fragment thereof having an immunological reactivity with a partial polypeptide of CAPRIN-1 protein, the partial polypeptide having the amino acid sequence shown by SEQ ID NO: 299 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 301, 302 and 303 (CDR1, CDR2 and CDR3, respectively) and a light-chain variable region comprising complementarity determining regions of SEQ ID NOs: 305, 306 and 307 (CDR1, CDR2 and CDR3, respectively), and having an immunological reactivity with CAPRIN-1 protein, and more preferably an antibody or a fragment thereof comprising a heavy-chain variable region comprising the amino acid sequence of SEQ ID NO: 300 and a light-chain variable region comprising the amino acid sequence of SEQ ID NO: 304.

An antibody or a fragment thereof having an immunological reactivity with a partial polypeptide of CAPRIN-1 protein, the partial polypeptide having the amino acid sequence shown by SEQ ID NO: 308 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 134, 135 and 136 (CDR1, CDR2 and CDR3, respectively) and a light-chain variable region comprising complementarity determining regions of SEQ ID NOs: 137, 138 and 139 (CDR1, CDR2 and CDR3, respectively), and having an immunological reactivity with CAPRIN-1 protein, and more preferably an antibody or a fragment thereof comprising a heavy-chain variable region comprising the amino acid sequence of SEQ ID NO: 68 and a light-chain variable region comprising the amino acid sequence of SEQ ID NO: 69.

An antibody or a fragment thereof having an immunological reactivity with a partial polypeptide of CAPRIN-1 protein, the partial polypeptide having the amino acid sequence shown by SEQ ID NO: 309 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 134, 135 and 136 (CDR1, CDR2 and CDR3, respectively) and a light-chain variable region comprising complementarity determining regions of SEQ ID NOs: 137, 138 and 139 (CDR1, CDR2 and CDR3, respectively), and having an immunological reactivity with CAPRIN-1 protein, and more preferably an antibody or a fragment thereof comprising a heavy-chain variable region comprising the amino acid sequence of SEQ ID NO: 68 and a light-chain variable region comprising the amino acid sequence of SEQ ID NO: 69.

In addition, the following anti-CAPRIN-1 antibodies are also preferably used.

An antibody or a fragment thereof comprising a heavy-chain variable region comprising the amino acid sequence of SEQ ID NO: 68 and a light-chain variable region comprising the amino acid sequence of SEQ ID NO: 69.

An antibody or a fragment thereof comprising a heavy-chain variable region comprising the amino acid sequence of SEQ ID NO: 70 and a light-chain variable region comprising the amino acid sequence of SEQ ID NO: 71.

An antibody or a fragment thereof comprising a heavy-chain variable region comprising the amino acid sequence of SEQ ID NO: 72 and a light-chain variable region comprising the amino acid sequence of SEQ ID NO: 73.

An antibody or a fragment thereof comprising a heavy-chain variable region comprising the amino acid sequence of SEQ ID NO: 74 and a light-chain variable region comprising the amino acid sequence of SEQ ID NO: 75.

An antibody or a fragment thereof comprising a heavy-chain variable region comprising the amino acid sequence of SEQ ID NO: 76 and a light-chain variable region comprising the amino acid sequence of SEQ ID NO: 77.

An antibody or a fragment thereof comprising a heavy-chain variable region comprising the amino acid sequence of SEQ ID NO: 78 and a light-chain variable region comprising the amino acid sequence of SEQ ID NO: 79.

An antibody or a fragment thereof comprising a heavy-chain variable region comprising the amino acid sequence of SEQ ID NO: 80 and a light-chain variable region comprising the amino acid sequence of SEQ ID NO: 81.

An antibody or a fragment thereof comprising a heavy-chain variable region comprising the amino acid sequence of SEQ ID NO: 82 and a light-chain variable region comprising the amino acid sequence of SEQ ID NO: 83.

An antibody or a fragment thereof comprising a heavy-chain variable region comprising the amino acid sequence of SEQ ID NO: 84 and a light-chain variable region comprising the amino acid sequence of SEQ ID NO: 85.

An antibody or a fragment thereof comprising a heavy-chain variable region comprising the amino acid sequence of SEQ ID NO: 86 and a light-chain variable region comprising the amino acid sequence of SEQ ID NO: 87.

An antibody or a fragment thereof comprising a heavy-chain variable region comprising the amino acid sequence of SEQ ID NO: 88 and a light-chain variable region comprising the amino acid sequence of SEQ ID NO: 89.

An antibody or a fragment thereof comprising a heavy-chain variable region comprising the amino acid sequence of SEQ ID NO: 90 and a light-chain variable region comprising the amino acid sequence of SEQ ID NO: 91.

An antibody or a fragment thereof comprising a heavy-chain variable region comprising the amino acid sequence of SEQ ID NO: 92 and a light-chain variable region comprising the amino acid sequence of SEQ ID NO: 93.

An antibody or a fragment thereof comprising a heavy-chain variable region comprising the amino acid sequence of SEQ ID NO: 94 and a light-chain variable region comprising the amino acid sequence of SEQ ID NO: 95.

An antibody or a fragment thereof comprising a heavy-chain variable region comprising the amino acid sequence of SEQ ID NO: 96 and a light-chain variable region comprising the amino acid sequence of SEQ ID NO: 97.

An antibody or a fragment thereof comprising a heavy-chain variable region comprising the amino acid sequence of SEQ ID NO: 98 and a light-chain variable region comprising the amino acid sequence of SEQ ID NO: 99.

An antibody or a fragment thereof comprising a heavy-chain variable region comprising the amino acid sequence of SEQ ID NO: 100 and a light-chain variable region comprising the amino acid sequence of SEQ ID NO: 101.

An antibody or a fragment thereof comprising a heavy-chain variable region comprising the amino acid sequence of SEQ ID NO: 102 and a light-chain variable region comprising the amino acid sequence of SEQ ID NO: 103.

An antibody or a fragment thereof comprising a heavy-chain variable region comprising the amino acid sequence of SEQ ID NO: 104 and a light-chain variable region comprising the amino acid sequence of SEQ ID NO: 105.

An antibody or a fragment thereof comprising a heavy-chain variable region comprising the amino acid sequence of SEQ ID NO: 106 and a light-chain variable region comprising the amino acid sequence of SEQ ID NO: 107.

An antibody or a fragment thereof comprising a heavy-chain variable region comprising the amino acid sequence of SEQ ID NO: 108 and a light-chain variable region comprising the amino acid sequence of SEQ ID NO: 109.

An antibody or a fragment thereof comprising a heavy-chain variable region comprising the amino acid sequence of SEQ ID NO: 110 and a light-chain variable region comprising the amino acid sequence of SEQ ID NO: 111.

An antibody or a fragment thereof comprising a heavy-chain variable region comprising the amino acid sequence of SEQ ID NO: 112 and a light-chain variable region comprising the amino acid sequence of SEQ ID NO: 113.

An antibody or a fragment thereof comprising a heavy-chain variable region comprising the amino acid sequence of SEQ ID NO: 114 and a light-chain variable region comprising the amino acid sequence of SEQ ID NO: 115.

An antibody or a fragment thereof comprising a heavy-chain variable region comprising the amino acid sequence of SEQ ID NO: 116 and a light-chain variable region comprising the amino acid sequence of SEQ ID NO: 117.

An antibody or a fragment thereof comprising a heavy-chain variable region comprising the amino acid sequence of SEQ ID NO: 118 and a light-chain variable region comprising the amino acid sequence of SEQ ID NO: 119.

An antibody or a fragment thereof comprising a heavy-chain variable region comprising the amino acid sequence of SEQ ID NO: 120 and a light-chain variable region comprising the amino acid sequence of SEQ ID NO: 121.

An antibody or a fragment thereof comprising a heavy-chain variable region comprising the amino acid sequence of SEQ ID NO: 122 and a light-chain variable region comprising the amino acid sequence of SEQ ID NO: 123.

An antibody or a fragment thereof comprising a heavy-chain variable region comprising the amino acid sequence of SEQ ID NO: 124 and a light-chain variable region comprising the amino acid sequence of SEQ ID NO: 125.

An antibody or a fragment thereof comprising a heavy-chain variable region comprising the amino acid sequence of SEQ ID NO: 126 and a light-chain variable region comprising the amino acid sequence of SEQ ID NO: 127.

An antibody or a fragment thereof comprising a heavy-chain variable region comprising the amino acid sequence of SEQ ID NO: 128 and a light-chain variable region comprising the amino acid sequence of SEQ ID NO: 129.

An antibody or a fragment thereof comprising a heavy-chain variable region comprising the amino acid sequence of SEQ ID NO: 130 and a light-chain variable region comprising the amino acid sequence of SEQ ID NO: 131.

An antibody or a fragment thereof comprising a heavy-chain variable region comprising the amino acid sequence of SEQ ID NO: 132 and a light-chain variable region comprising the amino acid sequence of SEQ ID NO: 133.

An antibody or a fragment thereof comprising a heavy-chain variable region comprising the amino acid sequence of SEQ ID NO: 300 and a light-chain variable region comprising the amino acid sequence of SEQ ID NO: 304.

In Examples mentioned later, the polyclonal antibody or the monoclonal antibody against a full-length CAPRIN-1 protein or a polypeptide of a portion of a region expressed on cell membrane surfaces of cancer cells was confirmed to exhibit reactivity with cell membrane surfaces of a plurality of human cancer cells. Furthermore, results indicating the response in human cancer patients were obtained, and a marked antitumor effect was obtained by which a tumor completely disappeared at some cancer sites.

### <PDGFR inhibitor>

The PDGFR inhibitor is a drug having the action of inhibiting the activity of platelet-derived growth factor receptor (PDGFR) mentioned below in detail.

PDGFR is a kind of receptor-type tyrosine kinase, and has two structurally similar subtypes, PDGFRα and PDGFRβ. The structures of both subtypes are characterized by having five extracellular immunoglobulin-like domains, and further having a single transmembrane domain, and an intracellular TK domain (ATP binding pocket and active loop) bisected with a kinase insertion sequence region necessary for binding effector molecules and adaptor molecules. Although these receptor proteins are usually present as monomers on cell membranes, they form dimers as a result of binding of ligands, and the tyrosine residues of PDGFR undergo autophosphorylation to provide a binding site for signaling molecules having a SH2 domain (PLC-γ, Grb2, PI3K, or the like), and downstream signaling pathways such as Ras-MAPK, PI3K-AKT, and PLγ-PKC are activated, thereby promoting migration, proliferation, cell survival, and the like. There are three kinds of combinations of dimers to be formed: PDGFR-αα, PDGFR-αβ, and PDGFR-ββ, and these complexes have different affinities for ligands, respectively. Aberrant expression of PDGFRα is known to be involved in abnormal development in vertebrate models and many diseases in humans. PDGFRα is widely expressed in kidney cells that are involved in processes of fibrosis and sclerosis. PDGF and its receptor PDGFRα are inducers of fibrosis in the convalescent phase of inflammatory bowel diseases and it has been suggested that they may also be involved in the active inflammatory phase. PDGFRβ is involved in the development of cardiovascular system, cell proliferation, and migration. Upon binding of a ligand platelet-derived growth factor (PDGF), PDGFRβ forms homodimers or heterodimers with PDGFRα to be activated, and activates downstream signals such as MAPK, PI3K/Akt/mTOR, JAK/STAT, PLCγ, and NF-κB. The fusion gene formed by translocation with ETV6 of chromosome 12 has been observed in chronic myeloproliferative diseases.

PDGFR inhibitors are known to exert a cancer therapeutic effect by inhibiting signaling pathways by PDGFR dimerization inhibition, ligand neutralization, ligand binding inhibition, internalization of PDGFR or the like, whereas the present invention can increase the drug efficacy of cancer immunotherapy itself, specifically, the phagocytic activity against cancer cells by immunocytes as shown in the Examples herein. Since the signaling pathway activated by PDGFR is not directly related to the immune system, the increase of the drug efficacy of cancer immunotherapy itself by the present invention is a new finding that cannot be expected from the technical level of a person skilled in the art at present.

Specific examples of the PDGFR inhibitor include agerafenib (RXDX-105), amuvatinib (MP-470), avapritinib (BLU-285), axitinib (AG 013736), Cediranib (AZD2171), crenolanib (CP-868596), dasatinib (BMS 345825/BMS 354825/BMS 35482513/Sprycel), erdafitinib (JNJ-42756493), flumatinib (HH-GV-678), foretinib (GSK1363089), imatinib (STI571), imatinib mesylate, lenvatinib (E7080), lenvatinib mesylate, linifanib (ABT-869), masitinib (AB1010), masitinib mesylate, motesanib diphosphate (AMG-706), nintedanib (BIBF 1120), nilotinib (AMN107/Tasigna), orantinib (SU6668), pazopanib (GW786034), pazopanib HCl (GW786034 HCl), ponatinib (AP24534), regorafenib (BAY 73-4506), regorafenib monohydrate, ripretinib (DCC-2618), seralutinib (GB002), sorafenib (BAY 43-9006), sorafenib tosylate, sunitinib (SU11248), telatinib, tivozanib (AV-951), toceranib phosphate, PDGFR inhibitor 1, X-82(Vorolanib), AZD2932, AZD3229, CP-673451, ENMD-2076, JNJ-10198409, Ki20227, Ki8751, MK-2461, N-(p-Coumaroyl) serotonin, ON123300, PP121, SKLB 610, SU14813, SU5402, trapidil, tyrphostin 9, tyrphostin AG 1296, and pharmaceutically acceptable (known) salts or (known) derivatives thereof. Among these PDGFR inhibitors, it is preferable that the PDGFR inhibitor is agerafenib, amuvatinib, avapritinib, axitinib, cediranib, crenolanib, dasatinib, erdafitinib, flumatinib, foretinib, imatinib, imatinib mesylate, lenvatinib, lenvatinib mesylate, linifanib, masitinib, masitinib mesylate, motesanib diphosphate (AMG-706), nintedanib, nilotinib, orantinib, pazopanib, pazopanib HCl (GW786034 HCl), ponatinib, regorafenib, regorafenib monohydrate, ripretinib, seralutinib, sorafenib, sorafenib tosylate, sunitinib, telatinib, tivozanib, or a pharmaceutically acceptable (known) salt or (known) derivative thereof, it is more preferable that the PDGFR inhibitor is sorafenib, sunitinib, dasatinib, nilotinib, pazopanib, regorafenib, nintedanib, or a pharmaceutically acceptable (known) salt or (known) derivative thereof; and it is further preferable that the PDGFR inhibitor is sorafenib or a pharmaceutically acceptable (known) salt or (known) derivative thereof.

Sorafenib (also known as BAY 43-9006, NSC-724772) inhibits the activity of PDGFR-β. The CAS number is shown by 284461-73-0, and the IUPAC name is shown by 4-[4-[[4-chloro-3-(trifluoromethyl)phenyl]carbamoylamino]phenoxy]-N-methylpyridine-2-carboxamide. The molecular formula is C₂₁H₁₆ClF₃N₄O₃, and the molecular weight is 464.8. Sorafenib is also used as sorafenib tosylate, where the CAS number is shown by 475207-59-1, and the IUPAC name is shown by 4-[4-[[4-chloro-3-(trifluoromethyl)phenyl]carbamoylamino]phenoxy]-N-methylpyridine-2-carboxamide;4-methylbenzenesulfonic acid. The molecular formula is C₂₈H₂₄ClF₃N₄O₆S, and the molecular weight is 637.0. Sorafenib simply described herein is a generic name for a free form and tosylate of sorafenib.

### <Other drugs>

The medicament of the present invention may comprise, as an active ingredient, an antitumor agent known in literatures and the like, in addition to the anti-CAPRIN-1 antibody and the PDGFR inhibitor, as long as it does not inhibit the effects as the medicament of the present invention. Specific examples of known antitumor agents include, but are not particularly limited to, 5-fluorouracil, irinotecan, oxaliplatin, carboplatin, cisplatin, nedaplatin, gemcitabine, paclitaxel, nabpaclitaxel, imiquimod, immune checkpoint inhibitors, doxorubicin, daunorubicin, cyclophosphamide, methotrexate, thiotepa, busulfan, improsulfan, piposulfan, benzodopa, carboquone, meturedopa, uredopa, altretamine, triethylenemelamine, triethylenephosphoramide, triethilenethiophosphoramide, trimethylolomelamine, bullatacin, bullatacinone, camptothecin, bryostatin, callystatin, cryptophycin 1, cryptophycin 8, dolastatin, duocarmycin, eleutherobin, pancratistatin, sarcodictyin, spongistatin, chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard, carmustine, chlorozotocin, fotemustine, lomustine, nimustine, ranimustine, calicheamicin, dynemicin, clodronate, esperamicin, aclacinomycin, actinomycin, authramycin, azaserine, bleomycin, cactinomycin, carabicin, carminomycin, carzinophilin, chromomycin, dactinomycin, detorbicin, 6-diazo-5-oxo-L-norleucine, adriamycin, epirubicin, esorubicin, idarubicin, marcellomycin, mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin, denopterin, pteropterin, trimetrexate, fludarabine, 6-mercaptopurine, thiamiprine, thioguanine, ancitabine, azacitidine, 6-azauridine, carmofur, cytarabine, dideoxyuridine, doxifluridine, enocitabine, floxuridine, androgens such as calusterone, dromostanolone propionate, epitiostanol, mepitiostane, testolactone, aminoglutethimide, mitotane, trilostane, frolinic acid, aceglatone, aldophosphamide glycoside, aminolevulinic acid, eniluracil, amsacrine, bestrabucil, bisantrene, defofamine, demecolcine, diaziquone, eflomithine, elliptinium acetate, epothilone, etoglucid, lentinan, lonidamine, maytansine, ansamitocine, mitoguazone, mitoxantrone, mopidanmol, nitraerine, pentostatin, phenamet, pirarubicin, losoxantrone, podophyllinic acid, 2-ethylhydrazide, procarbazine, razoxane, rhizoxin, schizophyllan, spirogermanium, tenuazonic acid, triaziquone, roridine A, anguidine, urethane, vindesine, dacarbazine, mannomustine, mitobronitol, mitolactol, pipobroman, gacytosine, docetaxel, gemcitabine, 6-thioguanine, mercaptopurine, vinblastine, etoposide, vincristine, vinorelbine, novantrone, teniposide, edatrexate, daunomycin, aminopterin, xeloda, ibandronate, difluoromethylornithine (DMFO), topoisomerase inhibitors, retinoic acid, folinic acid, and their pharmaceutically acceptable (known) salts or (known) derivatives.

### <Antitumor effect of present invention>

The antitumor effect of the combination of the anti-CAPRIN-1 antibody and the PDGFR inhibitor according to the present invention can be evaluated *in vivo* or *in vitro.* The *in vivo* antitumor effect can be evaluated by administering the anti-CAPRIN-1 antibody and the PDGFR inhibitor to an organism having cancer, measuring the size of a tumor after the administration, and examining the size of the cancer over time. Also, the *in vivo* antitumor effect can be evaluated by examining a survival rate of organisms. Alternatively, the *in vivo* antitumor effect may be evaluated by examining the ability to produce cytokines or chemokines. The *in vivo* antitumor effect can be further evaluated by examining prevention of cancer, prevention of metastasis or prevention of recurrence.

The *in vitro* antitumor effect can be evaluated by examining the cytotoxic activity or phagocytic activity against cancer cells by immunocytes in coculturing the cancer cells and the immunocytes. Thus, the antitumor effect of the combination of the anti-CAPRIN-1 antibody and the PDGFR inhibitor can be evaluated by adding the anti-CAPRIN-1 antibody and the PDGFR inhibitor in combination to a coculture system of cancer cells and immunocytes, and examining the cytotoxic activity or phagocytic activity against the cancer cells by the immunocytes. The immunocyte for use here may be any cell as long as the immunocyte is a blood cell having cytotoxic activity or phagocytic activity. A human NK cell is preferred for evaluating the cytotoxic activity, and a human monocytic cell (THP-1 or U937) is preferred for evaluating the phagocytic activity. When an antibody binds to a cancer cell, this is recognized by an immunocyte so that the cancer cell is killed via cytotoxic activity or phagocytic activity by the immunocyte. Therefore, an *in vivo* antitumor effect can be predicted by evaluating the *in vitro* antitumor effect.

An ability of an anti-CAPRIN-1 antibody used in the present invention to bind to CAPRIN-1 can be determined via binding assay using, for example, ELISA, a Western blot method, immunofluorescence, or flowcytometry analysis.

The combination of the anti-CAPRIN-1 antibody and the PDGFR inhibitor of the present invention increases an *in vitro* antitumor effect as compared with the anti-CAPRIN-1 antibody alone. The rate of increase is preferably 1.5 or more times, more preferably 2 or more times, and further preferably 3 or more times.

### <Medicament for treatment and/or prevention of cancer>

A medicament of the present invention is aimed at treating and/or preventing cancer. A cancer targeted by the medicament of the present invention is not particularly limited as long as it is a cancer (cells) expressing CAPRIN-1 protein, particularly, a cancer (cells) expressing CAPRIN-1 protein on a cell membrane surface.

The term "treatment" used herein refers to treatment of cancer based on an antitumor effect mentioned above. The term "prevention" used herein refers to not only prevention of development of cancer but also prevention of metastasis or recurrence of cancer.

Both the terms "tumor" and "cancer" used herein refer to malignant neoplasm, and thus they are used in an exchangeable manner.

Cancer that can be a target in the present invention is any cancer as long as the cancer expresses CAPRIN-1 protein on a cell membrane surface. The cancer is preferably colon cancer, lung cancer, prostate cancer, ovarian cancer, pancreatic cancer, kidney cancer, breast cancer, gastric cancer, bile duct cancer, thyroid cancer, melanoma, renal cell cancer, Hodgkin's lymphoma, head and neck cancer, mesothelioma, colorectal cancer, esophageal cancer, gastroesophageal junction cancer, hepatocellular cancer, glioblastoma, urothelial cancer, urinary bladder cancer, uterus cancer, primary central nervous system lymphoma, primary testicular lymphoma, biliary tract cancer, brain tumor, leukemia, liver cancer, sarcoma, fibrosarcoma, mastocytoma, adrenal cortex cancer, Ewing's tumor, testicle cancer, basal cell cancer, lymphoma, multiple myeloma, Paget's disease or skin cancer. These cancers may be primary cancers, metastatic cancers, cancers that have metastasized, cancers that have recurred, postoperative cancers, or unresectable cancers. The term melanoma is often used interchangeably with malignant melanoma.

More specifically, examples of the cancer include, but are not limited to, for example, cutaneous T/NK-cell lymphoma, peripheral T-cell lymphoma, multiple myeloma, Bowen's disease, prickle cell cancer, extramammary Paget's disease, mycosis fungoides, Sezary's syndrome, T-cell leukemia or lymphoma having a lesion only in the skin, cutaneous B-cell lymphoma (indolent group), cutaneous T-cell lymphatic or mammary adenoma, complex mammary adenoma, malignant mixed tumor of mammary gland, intraductal papillary carcinoma of mammary gland, lung adenocarcinoma, squamous cell cancer, small cell cancer, large cell cancer, glioma which is a neuroepithelial tissue tumor, glioblastoma, neuroblastoma, ependymoma, neuronal tumor, neuroectodermal tumor, neurilemoma, neurofibromatosis, meningioma, chronic lymphocytic leukemia, lymphoma, alimentary lymphoma, gastrointestinal lymphoma, small to medium cell lymphoma, cecal cancer, ascending colon cancer, descending colon cancer, transverse colon cancer, sigmoid colon cancer, rectal cancer, ovarian epithelial cancer, germ cell tumor, interstitial cell tumor, pancreatic duct cancer, invasive pancreatic duct cancer, adenocarcinoma of pancreatic cancer, acinar cell cancer, adenosquamous cancer, giant cell tumor, intraductal papillary mucinous tumor, mucinous cystadenocarcinoma, pancreatoblastoma, pancreatic islet cell tumor, Frantz's tumor, serous cystadenocarcinoma, solid pseudopapillary cancer, gastrinoma, glucagonoma, insulinoma, multiple endocrine neoplasia type-1 (Wermer syndrome), nonfunctional islet cell tumor, somatostatinoma, VIPoma, uterine cervical cancer, uterine body cancer, fibrosarcoma, osteosarcoma, joint sarcoma, Ewing sarcoma, Wilms's tumor, hepatoblastoma, soft tissue sarcoma, acute leukemia, chronic leukemia, spinal cord tumor, malignant soft tissue tumor, tumors of teratoma group, and head and neck cancer including hypopharynx cancer, oropharynx cancer, tongue cancer, nasopharyngeal cancer, oral cavity cancer, lip cancer, sinus cancer, voice box cancer, cancer of the renal pelvis and ureter, urinary bladder cancer, urethra cancer, testicular tumor, malignant pleural mesothelioma, malignant bone tumor, uterine body cancer, and pediatric malignant solid tumor (rhabdomyosarcoma, neuroblastoma, hepatoblastoma, medulloblastoma, nephroblastoma, retinoblastoma, central nervous system germ cell tumor, and Ewing sarcoma family of tumors). The cancer also includes a palpable cancer, a subcutaneously existing cancer, an intracutaneously existing cancer, a superficial cancer, cancer existing in the dermis, cancer existing in a non-parenchymal organ, and progressive caner which originate from the cancers described above. The cancer also includes a palpable cancer, a subcutaneously existing cancer, an intracutaneously existing cancer, a superficial cancer, cancer existing in the dermis, and cancer existing in a non-parenchymal organ, which originate from the cancers described above and have metastasized and recurred.

A preferable subject (patient) that can be a target is a mammal and is, for example, a mammal including primates, pet animals, livestock animals, and sport animals. Humans, dogs and cats are particularly preferable.

A medicament of the present invention can be formulated by a method known to persons skilled in the art. For instance, the medicament of the present invention can be parenterally used in the form of a parenteral injection of: an aseptic solution comprising water or a pharmacologically acceptable non-water solution; or a suspension liquid. For each formulation or pharmaceutical composition in the medicament of the present invention, the active ingredient (at least one of the anti-CAPRIN-1 antibody and the PDGFR inhibitor) may be appropriately combined with, for example, a pharmacologically acceptable carrier, medium or additive; specifically, sterilized water, physiological saline, an isotonic solution, a buffer (buffer solution, etc.), plant oil, an oily solution, an antioxidant, a solubilizer, an emulsifier, a suspension, a surfactant, a stabilizer, a fragrance, an excipient, or a binder, and preferably, may be formulated by mixing with them in a unit dosage form required for a generally acceptable pharmaceutical formulation. An amount of an active ingredient in a formulation is determined such that an appropriate dosage within an indicated range can be achieved.

An aseptic composition for injection can be prepared in accordance with general formulation practice using a vehicle such as distilled water for injection. An aqueous solution for injection purposes includes, for example, physiological saline or isotonic solutions comprising glucose and other adjuvants such as D-sorbitol, D-mannose, D-mannitol, and sodium chloride. Such solution may be used with an appropriate dissolution aid. Such dissolution aid includes, for example, alcohols such as ethanol and polyalcohol, such as propylene glycol, polyethylene glycol, or nonion surfactants such as polysorbate 80(TM) and HCO-60. Oily liquid includes, for example, sesame oil or soybean oil. Such oily liquid may be used in combination with a dissolution aid such as benzyl benzoate or benzyl alcohol. In addition, it may be mixed with a buffering agent such as a phosphate buffer solution or a sodium acetate buffer solution, a soothing agent such as procaine hydrochloride, a stabilizer such as benzyl alcohol or phenol, or an antioxidant. In general, a formulated injection solution is introduced into an adequate ample.

The above pharmaceutical composition is orally or parenterally administered. Preferably, it is parenterally administered. Specifically, dosage forms include injectable agents, intranasally-administered agents, transpulmonarily-administered agents, and percutaneously-administered agents. For example, injectable agents can be systemically or locally administered via intravenous injection, intramuscular injection, intraperitoneal injection, subcutaneous injection, or intratumoral injection. The percutaneously-administered agents include, for example, agents called liniments and external medicines. The external medicines include, for example, solid agents, solutions, sprays, ointments, creams, and gels.

The administration method can be appropriately selected depending on age, weight, gender, and symptoms of a patient. A single dose of a pharmaceutical composition comprising at least one of the anti-CAPRIN-1 antibody and the PDGFR inhibitor can be selected within a range of, for example, 0.0001 mg to 1000 mg per kg of body weight as the amount of each active ingredient. Alternatively, the dose of the active ingredient can be selected within a range of, for example, 0.001 to 100000 mg per patient's body, or 0.1 mg to 300 mg or 1 mg to 30 mg per kg of patient's body weight; however, it is not necessarily limited thereto. The dose and the administration method are changed depending on patient age, weight, gender, and symptoms. However, persons skilled in the art can appropriately select the dose and the method.

### <Administration method>

Treatment and/or prevention of cancer with a medicament for treatment and/or prevention of cancer of the present invention includes various modes, in addition to administration as a medicament mentioned above. For example, respective active ingredients in a medicament of the present invention can be administered simultaneously, concurrently, or individually in a staggered manner. As a specific example, active ingredients can be administered within a time interval up to approximately 3 weeks, i.e., the second active ingredient can be administered from immediately up to approximately 3 weeks after administration of the first active ingredient. These administrations may be carried out subsequently to a surgical procedure, or a surgical procedure may be carried out between the administrations of the first and second drugs. In addition, a medicament for treatment and/or prevention of cancer of the present invention may be administered according to a plurality of administration cycles. For example, in the case of carrying out simultaneous administration of respective active ingredients in a medicament for treatment and/or prevention of cancer of the present invention, a pharmaceutical composition comprising both active ingredients is administered once per approximately 2 days to approximately 3 weeks as one cycle. Then, this treatment cycle may be repeated, if necessary, according to the judgment of a physician in charge. Likewise, in the case of scheduling a formulation in a staggered manner, respective administration periods of individual agents are adjusted so as to span the same period. The interval between cycles can vary from 0 to 2 months. Respective doses of the active ingredients in the medicament for treatment and/or prevention of cancer of the present invention can be set in the same way as in the respective doses of the active ingredients in a pharmaceutical composition described above.

### <Pharmaceutical kit>

A medicament for treatment and/or prevention of cancer of the present invention may be in the form of a pharmaceutical kit. The pharmaceutical kit is a package for using active ingredients in the form of separate pharmaceutical compositions (formulations) in a method for treating and/or preventing cancer. The package may comprise an instruction for administering each of the active ingredients. The respective active ingredients in the pharmaceutical compositions for treatment and/or prevention of cancer contained in the pharmaceutical kit can be in the form of pharmaceutical compositions each formulated as described above such that the active ingredients can be administered together or separately. Further, the pharmaceutical kit comprises active ingredients in amounts sufficient for one or more doses such that the active ingredients can be administered according to the administration method described above.

### <Treatment and/or prevention method>

On the basis of the contents specifically described above, the present invention further provides a method for treating and/or preventing cancer, comprising administering the medicament of the present invention, or the anti-CAPRIN-1 antibody and the PDGFR inhibitor according to the present invention to a subject (patient). For example, the present invention further provides a method for treating and/or preventing cancer, comprising administering the medicament, etc. of the present invention to a subject (patient) suspected of having cancer. In this embodiment, for example, the anti-CAPRIN-1 antibody (antibody or fragment thereof) and the PDGFR inhibitor according to the present invention, and an optional antitumor agent contained in the medicament can be administered simultaneously or separately to the subject (patient).

### Examples

The present invention is hereafter described in detail with reference to the following examples, although the scope of the present invention is not limited thereto.

### (Example 1) Production of anti-CAPRIN-1 antibody

One hundred (100) µg of a human CAPRIN-1 recombinant protein produced according to Example 3 of WO2010/016526 was mixed with a MPL+TDM adjuvant (Sigma) in an amount equivalent to that of the antigen protein. The mixture was used as an antigen solution per mouse. The antigen solution was administered intraperitoneally to 6-week-old Balb/c mice (Japan SLC Inc.) and then further administered 3 times and 24 times every week for completion of immunization. A spleen was removed on day 3 after the final immunization and then ground between two sterilized glass slides. Spleen cells were obtained by repeating the following procedure three times: washing with PBS (-) (Nissui), centrifuging at 1500rpm for 10 minutes, and removing supernatant. The obtained spleen cells were mixed with mouse myeloma cell SP2/0 (purchased from ATCC) at a ratio of 10 : 1. The PEG solution prepared by mixing 200 µl of RPMI1640 medium containing 10% FBS heated at 37°C and 800 µl of PEG1500 (Boehringer) was added to the cells. The solution was incubated for 5 minutes for cell fusion. Centrifugation was performed at 1700 rpm for 5 minutes to remove supernatants. Cells were suspended in 150 ml of RPMI1640 medium (HAT selective medium) containing 15% FBS, to which 2% equivalent of HAT solution (Gibco) had been added, and then seeded onto fifteen 96-well plates (Nunc) at 100 µl per well. Cells were cultured for 7 days under conditions of 37°C and 5% CO₂, so that hybridomas resulting from fusion of spleen cells to myeloma cells were obtained. Hybridomas were selected using binding affinity to CAPRIN-1 protein of the antibody produced by the prepared hybridomas as an indicator. The CAPRIN-1 protein solution (1 µg/ml) was added at 100 µl per well of 96-well plates and then incubated at 4°C for 18 hours. After each well was washed 3 times with PBS-T, 0.5% Bovine Serum Albumin (BSA) solution (Sigma) was added at 400 µl per well, and then the plates were incubated at room temperature for 3 hours. The solution was removed and then each well was washed 3 times with 400 µl of PBS-T. Each culture supernatant of the hybridomas obtained above was added at 100 µl per well and then incubated at room temperature for 2 hours. After each well was washed 3 times with PBS-T, an HRP-labeled anti-mouse IgG (H+L) antibody (Invitrogen) diluted 5000-fold with PBS was added at 100 µl per well and then incubated at room temperature for 1 hour. After each well was washed 3 times with PBS-T, a TMB substrate solution (Thermo) was added at 100 µl per well and then incubated for 15-30 minutes, so that a color reaction was performed. After color development, 1 N sulfuric acid was added at 100 µl per well to stop the reaction. Absorbance at 450 nm and absorbance at 595 nm were measured using an absorption spectrometer. As a result, a plurality of hybridomas producing antibodies with high absorbances were selected. The selected hybridomas were added at 0.5 hybridomas per well of 96-well plates and then cultured. After 1 week, hybridomas forming single colonies in wells were observed. Cells in these wells were further cultured. Hybridomas were selected using binding affinity to CAPRIN-1 protein of the antibody produced by cloned hybridomas as an indicator. The CAPRIN-1 protein solution (1 µg/ml) was added at 100 µl per well of 96-well plates and then incubated at 4°C for 18 hours. Each well was washed 3 times with PBS-T, a 0.5% BSA solution was added at 400 µl per well, and then incubated at room temperature for 3 hours. The solution was removed and then each well was washed 3 times with 400 µl of PBS-T. Each culture supernatant of the hybridomas obtained above was added at 100 µl per well and then incubated at room temperature for 2 hours. Each well was washed 3 times with PBS-T, an HRP-labeled anti-mouse IgG (H+L) antibody (Invitrogen) diluted 5000-fold with PBS was added at 100 µl per well and then incubated at room temperature for 1 hour. Each well was washed 3 times with PBS-T, a TMB substrate solution (Thermo) was added at 100 µl per well and then incubated for 15-30 minutes, so that a color reaction was performed. After color development, 1 N sulfuric acid was added at 100 µl per well to stop the reaction. Absorbance at 450 nm and absorbance at 595 nm were measured using an absorption spectrometer. As a result, a plurality of mouse monoclonal antibodies exerting reactivity with CAPRIN-1 protein were obtained.

Reactivity of each monoclonal antibody with human cancer cells confirmed to express CAPRIN-1 protein on cell membrane surfaces was further confirmed by flow cytometry. A mouse IgG control antibody exhibiting no reactivity with the cancer cells was used as a negative control. As a result of confirmation, several monoclonal antibodies were obtained which had stronger fluorescence intensity against the cancer cells than that of the mouse IgG control antibody and reacted strongly with the cell membrane surfaces of the cancer cells expressing CAPRIN-1 on the cell membrane surfaces. From among them, a monoclonal antibody against CAPRIN-1 described in WO2013/125630, which was an antibody comprising the amino acid sequence of a heavy-chain variable region shown by SEQ ID NO: 114 and the amino acid sequence of a light-chain variable region shown by SEQ ID NO: 115, was selected as a monoclonal antibody exhibiting reactivity with CAPRIN-1 protein.

CDR1 to CDR3 of the heavy-chain variable region of the antibody selected were identified. A nucleotide sequence was designed so as to be able to express a heavy-chain variable region having framework regions comprising a human antibody sequence. This nucleotide sequence was inserted to a vector for mammalian expression having an insert of a human IgG1 heavy-chain constant region. Likewise, CDR1 to CDR3 of the light-chain variable region were identified. A nucleotide sequence was designed so as to be able to express a light-chain variable region having framework regions comprising a human antibody sequence. This nucleotide sequence was inserted to a vector for mammalian expression having an insert of a human IgG1 light-chain constant region. These two recombinant expression vectors were introduced to mammalian cells according to a general method and then a culture supernatant containing humanized monoclonal antibody #1 (humanized antibody #1) against CAPRIN-1 was obtained. The amino acid sequences of heavy chain CDR1, CDR2, and CDR3 of the humanized monoclonal antibody #1 are shown by SEQ ID NOs: 272, 273, and 274, respectively. The amino acid sequences of light chain CDR1, CDR2, and CDR3 of the humanized monoclonal antibody #1 are shown by SEQ ID NOs: 275, 276, and 277, respectively.

The obtained culture supernatant containing the obtained humanized anti-CAPRIN-1 monoclonal antibody #1 was purified using Hitrap Protein A Sepharose FF (GE Healthcare BioSciences) according to a general method, replaced with PBS (-), and filtered through a 0.22 µm filter (Millipore) for preparation of the filtrate.

The specific reactivity of the anti-CAPRIN-1 antibody to CAPRIN-1 protein was detected and confirmed by ELISA using CAPRIN-1 protein immobilized on a plate.

The reactivity of the anti-CAPRIN-1 antibody with cancer cells without permeation treatment of cell membranes was examined by flow cytometry to confirm that a portion of CAPRIN-1 protein was expressed on cell membrane surfaces of cancer cells as shown in Examples given below.

It was confirmed by flow cytometry that, against all of breast cancer cells (BT-474), colon cancer cells (HT-29 and HCT116), lung cancer cells (QG56, H1650, and A549), gastric cancer cells (NCI-N87), uterus cancer cells (HEC-1-A), prostate cancer cells (22Rv1 and DU145), pancreatic cancer cells (Panc10.5), liver cancer cells (Hep3B), ovarian cancer cells (SKOV3), kidney cancer cells (Caki-2), brain tumor cells (U-87MG), urinary bladder cancer cells (T24), esophageal cancer cells (OE33), leukemia cells (OCI-AML5), lymphoma cells (Ramos), gallbladder cancer cells (TGBC14TKB), fibrosarcoma cells (HT-1080), and melanoma cells (G-361 and A375), which are human cancer cells confirmed to express CAPRIN-1 gene, and mouse kidney cancer cells (Renca) and mouse breast cancer cells (4T1) confirmed to express CAPRIN-1 gene, the humanized antibody #1 had stronger fluorescence intensity than that of a human IgG control antibody and rabbit IgG antibody serving as negative controls exhibiting no reactivity with the cancer cells, and reacted strongly with the cell membrane surfaces of the cancer cells expressing CAPRIN-1.

Likewise, the anti-CAPRIN-1 antibodies described in WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125640, WO2013/147169, WO2013/147176 and WO2015/020212 were also confirmed to react strongly with the cancer cell membrane surfaces.

### (Example 2A) In vitro antitumor effect 1 of combination of anti-CAPRIN-1 antibody and sorafenib

Antitumor effects of a combination of an anti-CAPRIN-1 antibody and a PDGFR inhibitor sorafenib were evaluated *in vitro.* The sorafenib used was sorafenib tosylate purchased from ChemScene LLC (Cat. No.: CS-0164).

Specifically, in PDGFR inhibitor combination test group, the drug used in combination was applied to human cancer cells, and then the cancer cells were cocultured with human monocytic cells (THP-1) in the presence of the anti-CAPRIN-1 antibody, and the antibody-mediated phagocytic activity against the cancer cells by THP-1 was evaluated.

The human-derived cancer cells used were a colon cancer cell line HCT116. On a 6-well plate, the human-derived cancer cells were cultured for 2 days in the presence of the sorafenib (10 µM), for the sorafenib combination test group. As a control, a test group without a drug used in combination, in which only the anti-CAPRIN-1 antibody was added, was established. In the control group, the cancer cells were cultured for 2 days without addition of the drug used in combination, beforehand.

Each cancer cell line after the culture was dissociated with TrypLE Express (Thermo), and the cancer cells were stained by the addition of calcein-AM at a final concentration of 0.04 µg/mL and incubation at 37°C for 10 minutes. Next, the cells were dispensed at 5 × 10³ cancer cells per well to a 96-well plate, and the anti-CAPRIN-1 antibody (final concentration: 1 µg/mL) and 1.25 × 10⁵ THP-1 cells were added to each well, followed by culture at 37°C for 1 hours under a condition of 5% CO₂. Then, the cells were washed with PBS (phosphate buffer solution) containing 1% FBS (fetal bovine serum), and the human monocytic cells were stained through a reaction with an APC (allophycocyanin)-labeled anti-human CD45 antibody (final concentration: 0.25 µg/mL). Further, dead cells were stained with propidium iodide (PI) (final concentration: 0.1 µg/mL), followed by the measurement of fluorescence from the cells by flow cytometry. PI-positive dead cells were excluded from analysis. The human monocytic cells THP-1 recognize an antibody bound onto cancer cells and phagocytize the cancer cells. At that time, if the cancer cells have been stained with calcein-AM, the monocytic cells ingesting the cancer cells by phagocytosis also become positive to calcein-AM. Thus, the phagocytic activity in this evaluation system was calculated as the ratio (%) of APC-positive/calcein-AM-positive cells to the whole calcein-AM-positive population.

As a result of evaluation using the anti-CAPRIN-1 antibody prepared in Example 1 (anti-CAPRIN-1 humanized antibody #1) as an anti-CAPRIN-1 antibody, phagocytic activity against cancer cells of 90% or more was observed for sorafenib combination test group, while phagocytic activity against cancer cells of 39% or less was observed against HCT116 for the test group without a drug used in combination. As a result of conducting Student's t test, the phagocytic activity of the sorafenib combination test group was significantly higher than that of the test group without a drug used in combination (p < 0.001; significance level: 5%) (Figure 1). Thus, the antitumor effect of the anti-CAPRIN-1 antibody on the human cancer cells was significantly increased by approximately 2.3 times by using the anti-CAPRIN-1 antibody in combination with sorafenib.

Combinations of any anti-CAPRIN-1 antibody described in WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125640, WO2013/147169, WO2013/147176 and WO2015/020212 and sorafenib exhibit similar increase in phagocytic activity against cancer cells as in the combination of the anti-CAPRIN-1 humanized antibody #1 and sorafenib against the above human cancer cells.

### (Example 2B) In vitro antitumor effect 2 of combination of anti-CAPRIN-1 antibody and sorafenib

Antitumor effects of a combination of an anti-CAPRIN-1 antibody and a PDGFR inhibitor sorafenib were evaluated *in vitro.* The sorafenib used was sorafenib tosylate purchased from ChemScene LLC (Cat. No.: CS-0164).

Specifically, in PDGFR inhibitor combination test group, the drug used in combination was applied to human cancer cells, and then the cancer cells were cocultured with human monocytic cells (THP-1) in the presence of the anti-CAPRIN-1 antibody, and the antibody-mediated phagocytic activity against the cancer cells by THP-1 was evaluated. A human IgG antibody (Sigma-Aldrich Co. LLC, Cat. No.: 4506) was used as a control antibody.

The human-derived cancer cell used was a colon cancer cell line HCT116. On a 6-well plate, the human-derived cancer cells were cultured for 2 days in the presence of the sorafenib (10µM), for the sorafenib combination test group. As a control, a test group without a drug used in combination, in which only the anti-CAPRIN-1 antibody was added, was established. In the control group, the cancer cells were cultured for 2 days without addition of the drug used in combination, beforehand.

Each cancer cell line after the culture was dissociated with TrypLE Express (Thermo), and the cancer cells were stained by the addition of calcein-AM at a final concentration of 0.04 µg/mL and incubation at 37°C for 10 minutes. Next, the cells were dispensed at 5 × 10³ cancer cells per well to a 96-well plate, and the anti-CAPRIN-1 antibody at a final concentration of 1 µg/mL (or control IgG antibody at the same concentration) and 1.25 × 10⁵ THP-1 cells were added to each well, followed by culture at 37°C for 1 hour under a condition of 5% CO₂. Then, the cells were washed with PBS (phosphate buffer solution) containing 1% FBS (fetal bovine serum), and the human monocytic cells were stained through a reaction with an APC (allophycocyanin)-labeled anti-human CD45 antibody (final concentration: 0.25 µg/mL). Further, dead cells were stained with propidium iodide (PI) (final concentration: 0.1 µg/mL), followed by the measurement of fluorescence from each cell by flow cytometry. PI-positive dead cells were excluded from analysis. The human monocytic cells THP-1 recognize an antibody bound onto cancer cells and phagocytize the cancer cells. At that time, if the cancer cells have been stained with calcein-AM, the monocytic cells ingesting the cancer cells by phagocytosis also become positive to calcein-AM. Thus, the phagocytic activity in this evaluation system was calculated as the ratio (%) of APC-positive/calcein-AM-positive cells to the whole calcein-AM-positive population.

As a result of evaluation using the anti-CAPRIN-1 antibody prepared in Example 1 (anti-CAPRIN-1 humanized antibody #1) as an anti-CAPRIN-1 antibody, phagocytic activity against cancer cells of 90% or more was observed for sorafenib combination test group, while phagocytic activity against cancer cells of 39% or less was observed against HCT116 for the test group without a drug used in combination. As a result of conducting Dunnett's test, the phagocytic activity of the sorafenib combination test group was significantly higher than that of the test group without a drug used in combination (p < 0.001; significance level: 5%) (Figure 2). Thus, the antitumor effect of the anti-CAPRIN-1 antibody on the human cancer cells was significantly increased by approximately 2.3 times by using the anti-CAPRIN-1 antibody in combination with sorafenib.

### (Example 3) In vitro antitumor effects of antibody alone or combination of antibody and sorafenib

Antitumor effects of anti-CAPRIN-1 antibody, anti-CD20 antibody or anti-HER-2 antibody each alone, or a combination of each of these antibodies and sorafenib were evaluated *in vitro.* The sorafenib used was sorafenib tosylate purchased from ChemScene LLC (Cat. No.: CS-0164). The anti-CD20 antibody ("Rituxan") and anti-HER-2 antibody ("Herceptin") used were purchased from Chugai Pharmaceutical Co., Ltd.

Specifically, in sorafenib combination test group, sorafenib was applied to human cancer cells, and then the cancer cells were cocultured with human monocytic cells (THP-1) in the presence of the anti-CAPRIN-1 antibody, anti-CD20 antibody or anti-HER-2 antibody at 1 µM, and the antibody-mediated phagocytic activity against the cancer cells by THP-1 was evaluated.

The human-derived cancer cell used was a colon cancer cell line HCT116. On a 6-well plate, the human-derived cancer cells were cultured for 2 days in the presence of the respective drugs used in combination, for the sorafenib combination test group. Specifically, sorafenib was added at a concentration of 10 µM to HCT 116. As a control, a test group without a drug used in combination, in which only the anti-CAPRIN-1 antibody, anti-CD20 antibody or anti-HER-2 antibody was added, was established. In the control group, the cancer cells were cultured for 2 days without addition of the sorafenib, beforehand.

Each cancer cell line after the culture was dissociated with TrypLE Express (Thermo), and the cancer cells were stained by the addition of calcein-AM at a final concentration of 0.04 µg/mL and incubation at 37°C for 10 minutes. Next, the cells were dispensed at 1 × 10⁴ cancer cells per well to a 96-well plate, and the anti-CAPRIN-1 antibody, anti-CD20 antibody or anti-HER-2 antibody at a final concentration of 1 µg/mL and 10 × 10⁵ THP-1 cells were added to each well, followed by culture at 37°C for 1 hour under a condition of 5% CO₂. Then, the cells were washed with PBS (phosphate buffer solution) containing 1% FBS (fetal bovine serum), and the human monocytic cells were stained through a reaction with an APC (allophycocyanin)-labeled anti-human CD45 antibody (final concentration: 0.25 µg/mL). Further, dead cells were stained with propidium iodide (PI) (final concentration: 0.1 µg/mL), followed by the measurement of fluorescence from each cell by flow cytometry. PI-positive dead cells were excluded from analysis. The human monocytic cells THP-1 recognize an antibody bound onto cancer cells and phagocytize the cancer cells. At that time, if the cancer cells have been stained with calcein-AM, the monocytic cells ingesting the cancer cells by phagocytosis also become positive to calcein-AM. Thus, the phagocytic activity in this evaluation system was calculated as the ratio (%) of APC-positive/calcein-AM-positive cells to the whole calcein-AM-positive population.

As a result of evaluation using the anti-CAPRIN-1 antibody prepared in Example 1 (anti-CAPRIN-1 humanized antibody #1) as an anti-CAPRIN-1 antibody, phagocytic activity against cancer cells of 64% or more was observed for sorafenib combination test group, while phagocytic activity against cancer cells of 21% or less was observed against HCT116 for the test group without a drug used in combination. As a result of conducting Dunnett's test, the phagocytic activity of the sorafenib combination test group was significantly higher than that of the test group without a drug used in combination (p < 0.001, significance level: 5%) (Figure 2).

On the other hand, as a result of evaluation using the anti-CD20 antibody or anti-HER-2 antibody, when the anti-CD20 antibody was used, phagocytic activity against cancer cells of 8% or less was observed for sorafenib combination test group, while phagocytic activity against cancer cells of 7% or less was observed against HCT116 for the test group without a drug used in combination, thus the drug efficacy was not significantly increased by the combination. When the anti-HER-2 antibody was used, phagocytic activity against cancer cells of 9% or less was observed for sorafenib combination test group, while phagocytic activity against cancer cells of 10% or less was observed against HCT116 for the test group without a drug used in combination, thus the drug efficacy was not significantly increased by the combination (Figure 3).

From the above, it has been suggested that the increase of the anti-tumor effect by the combination of the antibody and sorafenib is an anti-CAPRIN-1 antibody-specific effect.

Combinations of any anti-CAPRIN-1 antibody described in WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125640, WO2013/147169, WO2013/147176 and WO2015/020212 and each drug exhibit similar increase in phagocytic activity as in the anti-CAPRIN-1 humanized antibody #1 against the above human cancer cells.

### (Example 4) In vitro antitumor effect of combination of anti-CAPRIN-1 antibody and sorafenib or cisplatin

Antitumor effects of a combination of an anti-CAPRIN-1 antibody and sorafenib or cisplatin were evaluated *in vitro.* The sorafenib used was sorafenib tosylate purchased from ChemScene LLC (Cat. No.: CS-0164). The cisplatin used was purchased from Pfizer Inc.

Specifically, in each inhibitor combination test group, the indicated drug used in combination was applied to human cancer cells, and then the cancer cells were cocultured with human monocytic cells (THP-1) in the presence of the anti-CAPRIN-1 antibody, and the antibody-mediated phagocytic activity against the cancer cells by THP-1 was evaluated.

The human-derived cancer cell used was a colon cancer cell line HCT116. On a 6-well plate, the human-derived cancer cells were cultured for 2 days in the presence of the respective drugs used in combination, for the sorafenib combination test group. Specifically, sorafenib was added at a concentration of 10 µM and cisplatin at a concentration of 1 µM to HCT 116. As a control, a test group without a drug used in combination, in which only the anti-CAPRIN-1 antibody was added, was established. In the control group, the cancer cells were cultured for 2 days without addition of the drug used in combination, beforehand.

Each cancer cell line after the culture was dissociated with TrypLE Express (Thermo), and the cancer cells were stained by the addition of calcein-AM at a final concentration of 0.04 µg/mL and incubation at 37°C for 10 minutes. Next, the cells were dispensed at 1 × 10⁴ cancer cells per well to a 96-well plate, and the anti-CAPRIN-1 antibody at a final concentration of 1 µg/mL and 10 × 10⁵ THP-1 cells were added to each well, followed by culture at 37°C for 1 hour under a condition of 5% CO₂. Then, the cells were washed with PBS (phosphate buffer solution) containing 1% FBS (fetal bovine serum), and the human monocytic cells were stained through a reaction with an APC (allophycocyanin)-labeled anti-human CD45 antibody (final concentration: 0.25 µg/mL). Further, dead cells were stained with propidium iodide (PI) (final concentration: 0.1 µg/mL), followed by the measurement of fluorescence from each cell by flow cytometry. PI-positive dead cells were excluded from analysis. The human monocytic cells THP-1 recognize an antibody bound onto cancer cells and phagocytize the cancer cells. At that time, if the cancer cells have been stained with calcein-AM, the monocytic cells ingesting the cancer cells by phagocytosis also become positive to calcein-AM. Thus, the phagocytic activity in this evaluation system was calculated as the ratio (%) of APC-positive/calcein-AM-positive cells to the whole calcein-AM-positive population.

As a result of evaluation using the anti-CAPRIN-1 antibody prepared in Example 1 (anti-CAPRIN-1 humanized antibody #1) as an anti-CAPRIN-1 antibody, phagocytic activity against cancer cells of 64% or more was observed for sorafenib combination test group, while phagocytic activity against cancer cells of 21% or less was observed against HCT116 for the test group without a drug used in combination. On the other hand, phagocytic activity against cancer cells of 23% or less was observed for the cisplatin combination test group. As a result of conducting Dunnett's test, the phagocytic activity of the sorafenib combination test group was significantly higher than that of the test group without a drug used in combination (p < 0.001, significance level: 5%), while there was no significant change in the phagocytic activity of the cisplatin combination test group compared to that of the test group without a drug used in combination (Figure 4).

From the above, it has been suggested that the increase of anti-tumor effect by the drug combination is a PDGFR inhibitor-specific effect.

Combinations of any anti-CAPRIN-1 antibody described in WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125640, WO2013/147169, WO2013/147176 and WO2015/020212 and each drug exhibit similar change in phagocytic activity as in the anti-CAPRIN-1 humanized antibody #1 against the above human cancer cells.

All publications, patents and patent applications cited herein are incorporated herein by reference in their entirety.

## Claims

1. A medicament for treatment and/or prevention of cancer, comprising an antibody or a fragment thereof having an immunological reactivity with CAPRIN-1 protein, and a PDGFR inhibitor together or separately in combination.

2. The medicament according to claim 1, wherein the PDGFR inhibitor is sorafenib.

3. The medicament according to claim 1 or 2, wherein the antibody or the fragment thereof has an immunological reactivity with CAPRIN-1 protein having an amino acid sequence shown by any one of the even numbered SEQ ID NOs: 2 to 30, or an amino acid sequence having 80% or more sequence identity with the amino acid sequence.

4. The medicament according to any one of claims 1 to 3, wherein the antibody or the fragment thereof has an immunological reactivity with an extracellular region of CAPRIN-1 protein present on a cancer cell surface.

5. The medicament according to any one of claims 1 to 4, wherein the antibody or the fragment thereof has an immunological reactivity with a partial polypeptide of CAPRIN-1 protein, the partial polypeptide having an amino acid sequence shown by any one of SEQ ID NOs: 31 to 35, 296 to 299, 308 and 309, or an amino acid sequence having 80% or more sequence identity with the amino acid sequence.

6. The medicament according to any one of claims 1 to 5, wherein the antibody is a monoclonal antibody or a polyclonal antibody.

7. The medicament according to any one of claims 1 to 6, wherein the antibody or the fragment thereof is any one of the following (A) to (M):
(A) an antibody or a fragment thereof comprising a heavy-chain variable region comprising complementarity determining regions of SEQ ID NOs: 36, 37 and 38 (CDR1, CDR2 and CDR3, respectively) and a light-chain variable region comprising complementarity determining regions of SEQ ID NOs: 40, 41 and 42 (CDR1, CDR2 and CDR3, respectively), and having an immunological reactivity with CAPRIN-1 protein;
(B) an antibody or a fragment thereof comprising a heavy-chain variable region comprising complementarity determining regions of SEQ ID NOs: 44, 45 and 46 (CDR1, CDR2 and CDR3, respectively) and a light-chain variable region comprising complementarity determining regions of SEQ ID NOs: 48, 49 and 50 (CDR1, CDR2 and CDR3, respectively), and having an immunological reactivity with CAPRIN-1 protein;
(C) an antibody or a fragment thereof comprising a heavy-chain variable region comprising complementarity determining regions of SEQ ID NOs: 52, 53 and 54 (CDR1, CDR2 and CDR3, respectively) and a light-chain variable region comprising complementarity determining regions of SEQ ID NOs: 56, 57 and 58 (CDR1, CDR2 and CDR3, respectively), and having an immunological reactivity with CAPRIN-1 protein;
(D) an antibody or a fragment thereof comprising a heavy-chain variable region comprising complementarity determining regions of SEQ ID NOs: 60, 61 and 62 (CDR1, CDR2 and CDR3, respectively) and a light-chain variable region comprising complementarity determining regions of SEQ ID NOs: 64, 65 and 66 (CDR1, CDR2 and CDR3, respectively), and having an immunological reactivity with CAPRIN-1 protein;
(E) an antibody or a fragment thereof comprising a heavy-chain variable region comprising complementarity determining regions of SEQ ID NOs: 170, 171 and 172 (CDR1, CDR2 and CDR3, respectively) and a light-chain variable region comprising complementarity determining regions of SEQ ID NOs: 173, 174 and 175 (CDR1, CDR2 and CDR3, respectively), and having an immunological reactivity with CAPRIN-1 protein;
(F) an antibody or a fragment thereof comprising a heavy-chain variable region comprising complementarity determining regions of SEQ ID NOs: 176, 177 and 178 (CDR1, CDR2 and CDR3, respectively) and a light-chain variable region comprising complementarity determining regions of SEQ ID NOs: 179, 180 and 181 (CDR1, CDR2 and CDR3, respectively), and having an immunological reactivity with CAPRIN-1 protein;
(G) an antibody or a fragment thereof comprising a heavy-chain variable region comprising complementarity determining regions of SEQ ID NOs: 182, 183 and 184 (CDR1, CDR2 and CDR3, respectively) and a light-chain variable region comprising complementarity determining regions of SEQ ID NOs: 185, 186 and 187 (CDR1, CDR2 and CDR3, respectively), and having an immunological reactivity with CAPRIN-1 protein;
(H) an antibody or a fragment thereof comprising a heavy-chain variable region comprising complementarity determining regions of SEQ ID NOs: 188, 189 and 190 (CDR1, CDR2 and CDR3, respectively) and a light-chain variable region comprising complementarity determining regions of SEQ ID NOs: 191, 192 and 193 (CDR1, CDR2 and CDR3, respectively), and having an immunological reactivity with CAPRIN-1 protein;
(I) an antibody or a fragment thereof comprising a heavy-chain variable region comprising complementarity determining regions of SEQ ID NOs: 146, 147 and 148 (CDR1, CDR2 and CDR3, respectively) and a light-chain variable region comprising complementarity determining regions of SEQ ID NOs: 149, 150 and 151 (CDR1, CDR2 and CDR3, respectively), and having an immunological reactivity with CAPRIN-1 protein;
(J) an antibody or a fragment thereof comprising a heavy-chain variable region comprising complementarity determining regions of SEQ ID NOs: 272, 273 and 274 (CDR1, CDR2 and CDR3, respectively) and a light-chain variable region comprising complementarity determining regions of SEQ ID NOs: 275, 276 and 277 (CDR1, CDR2 and CDR3, respectively), and having an immunological reactivity with CAPRIN-1 protein;
(K) an antibody or a fragment thereof comprising a heavy-chain variable region comprising complementarity determining regions of SEQ ID NOs: 290, 291 and 292 (CDR1, CDR2 and CDR3, respectively) and a light-chain variable region comprising complementarity determining regions of SEQ ID NOs: 293, 294 and 295 (CDR1, CDR2 and CDR3, respectively), and having an immunological reactivity with CAPRIN-1 protein;
(L) an antibody or a fragment thereof comprising a heavy-chain variable region comprising complementarity determining regions of SEQ ID NOs: 301, 302 and 303 (CDR1, CDR2 and CDR3, respectively) and a light-chain variable region comprising complementarity determining regions of SEQ ID NOs: 305, 306 and 307 (CDR1, CDR2 and CDR3, respectively), and having an immunological reactivity with CAPRIN-1 protein; and
(M) an antibody or a fragment thereof comprising a heavy-chain variable region comprising complementarity determining regions of SEQ ID NOs: 134, 135 and 136 (CDR1, CDR2 and CDR3, respectively) and a light-chain variable region comprising complementarity determining regions of SEQ ID NOs: 137, 138 and 139 (CDR1, CDR2 and CDR3, respectively), and having an immunological reactivity with CAPRIN-1 protein.

8. The medicament according to any one of claims 1 to 7, wherein the antibody or the fragment thereof is any one of the following (a) to (al):
(a) an antibody or a fragment thereof comprising a heavy-chain variable region comprising the amino acid sequence of SEQ ID NO: 39 and a light-chain variable region comprising the amino acid sequence of SEQ ID NO: 43;
(b) an antibody or a fragment thereof comprising a heavy-chain variable region comprising the amino acid sequence of SEQ ID NO: 47 and a light-chain variable region comprising the amino acid sequence of SEQ ID NO: 51;
(c) an antibody or a fragment thereof comprising a heavy-chain variable region comprising the amino acid sequence of SEQ ID NO: 55 and a light-chain variable region comprising the amino acid sequence of SEQ ID NO: 59;
(d) an antibody or a fragment thereof comprising a heavy-chain variable region comprising the amino acid sequence of SEQ ID NO: 63 and a light-chain variable region comprising the amino acid sequence of SEQ ID NO: 67;
(e) an antibody or a fragment thereof comprising a heavy-chain variable region comprising the amino acid sequence of SEQ ID NO: 68 and a light-chain variable region comprising the amino acid sequence of SEQ ID NO: 69;
(f) an antibody or a fragment thereof comprising a heavy-chain variable region comprising the amino acid sequence of SEQ ID NO: 70 and a light-chain variable region comprising the amino acid sequence of SEQ ID NO: 71;
(g) an antibody or a fragment thereof comprising a heavy-chain variable region comprising the amino acid sequence of SEQ ID NO: 72 and a light-chain variable region comprising the amino acid sequence of SEQ ID NO: 73;
(h) an antibody or a fragment thereof comprising a heavy-chain variable region comprising the amino acid sequence of SEQ ID NO: 74 and a light-chain variable region comprising the amino acid sequence of SEQ ID NO: 75;
(i) an antibody or a fragment thereof comprising a heavy-chain variable region comprising the amino acid sequence of SEQ ID NO: 76 and a light-chain variable region comprising the amino acid sequence of SEQ ID NO: 77;
(j) an antibody or a fragment thereof comprising a heavy-chain variable region comprising the amino acid sequence of SEQ ID NO: 78 and a light-chain variable region comprising the amino acid sequence of SEQ ID NO: 79;
(k) an antibody or a fragment thereof comprising a heavy-chain variable region comprising the amino acid sequence of SEQ ID NO: 80 and a light-chain variable region comprising the amino acid sequence of SEQ ID NO: 81;
(l) an antibody or a fragment thereof comprising a heavy-chain variable region comprising the amino acid sequence of SEQ ID NO: 82 and a light-chain variable region comprising the amino acid sequence of SEQ ID NO: 83;
(m) an antibody or a fragment thereof comprising a heavy-chain variable region comprising the amino acid sequence of SEQ ID NO: 84 and a light-chain variable region comprising the amino acid sequence of SEQ ID NO: 85;
(n) an antibody or a fragment thereof comprising a heavy-chain variable region comprising the amino acid sequence of SEQ ID NO: 86 and a light-chain variable region comprising the amino acid sequence of SEQ ID NO: 87;
(o) an antibody or a fragment thereof comprising a heavy-chain variable region comprising the amino acid sequence of SEQ ID NO: 88 and a light-chain variable region comprising the amino acid sequence of SEQ ID NO: 89;
(p) an antibody or a fragment thereof comprising a heavy-chain variable region comprising the amino acid sequence of SEQ ID NO: 90 and a light-chain variable region comprising the amino acid sequence of SEQ ID NO: 91;
(q) an antibody or a fragment thereof comprising a heavy-chain variable region comprising the amino acid sequence of SEQ ID NO: 92 and a light-chain variable region comprising the amino acid sequence of SEQ ID NO: 93;
(r) an antibody or a fragment thereof comprising a heavy-chain variable region comprising the amino acid sequence of SEQ ID NO: 94 and a light-chain variable region comprising the amino acid sequence of SEQ ID NO: 95;
(s) an antibody or a fragment thereof comprising a heavy-chain variable region comprising the amino acid sequence of SEQ ID NO: 96 and a light-chain variable region comprising the amino acid sequence of SEQ ID NO: 97;
(t) an antibody or a fragment thereof comprising a heavy-chain variable region comprising the amino acid sequence of SEQ ID NO: 98 and a light-chain variable region comprising the amino acid sequence of SEQ ID NO: 99;
(u) an antibody or a fragment thereof comprising a heavy-chain variable region comprising the amino acid sequence of SEQ ID NO: 100 and a light-chain variable region comprising the amino acid sequence of SEQ ID NO: 101;
(v) an antibody or a fragment thereof comprising a heavy-chain variable region comprising the amino acid sequence of SEQ ID NO: 102 and a light-chain variable region comprising the amino acid sequence of SEQ ID NO: 103;
(w) an antibody or a fragment thereof comprising a heavy-chain variable region comprising the amino acid sequence of SEQ ID NO: 104 and a light-chain variable region comprising the amino acid sequence of SEQ ID NO: 105;
(x) an antibody or a fragment thereof comprising a heavy-chain variable region comprising the amino acid sequence of SEQ ID NO: 106 and a light-chain variable region comprising the amino acid sequence of SEQ ID NO: 107;
(y) an antibody or a fragment thereof comprising a heavy-chain variable region comprising the amino acid sequence of SEQ ID NO: 108 and a light-chain variable region comprising the amino acid sequence of SEQ ID NO: 109;
(z) an antibody or a fragment thereof comprising a heavy-chain variable region comprising the amino acid sequence of SEQ ID NO: 110 and a light-chain variable region comprising the amino acid sequence of SEQ ID NO: 111;
(aa) an antibody or a fragment thereof comprising a heavy-chain variable region comprising the amino acid sequence of SEQ ID NO: 112 and a light-chain variable region comprising the amino acid sequence of SEQ ID NO: 113;
(ab) an antibody or a fragment thereof comprising a heavy-chain variable region comprising the amino acid sequence of SEQ ID NO: 114 and a light-chain variable region comprising the amino acid sequence of SEQ ID NO: 115;
(ac) an antibody or a fragment thereof comprising a heavy-chain variable region comprising the amino acid sequence of SEQ ID NO: 116 and a light-chain variable region comprising the amino acid sequence of SEQ ID NO: 117;
(ad) an antibody or a fragment thereof comprising a heavy-chain variable region comprising the amino acid sequence of SEQ ID NO: 118 and a light-chain variable region comprising the amino acid sequence of SEQ ID NO: 119;
(ae) an antibody or a fragment thereof comprising a heavy-chain variable region comprising the amino acid sequence of SEQ ID NO: 120 and a light-chain variable region comprising the amino acid sequence of SEQ ID NO: 121;
(af) an antibody or a fragment thereof comprising a heavy-chain variable region comprising the amino acid sequence of SEQ ID NO: 122 and a light-chain variable region comprising the amino acid sequence of SEQ ID NO: 123;
(ag) an antibody or a fragment thereof comprising a heavy-chain variable region comprising the amino acid sequence of SEQ ID NO: 124 and a light-chain variable region comprising the amino acid sequence of SEQ ID NO: 125;
(ah) an antibody or a fragment thereof comprising a heavy-chain variable region comprising the amino acid sequence of SEQ ID NO: 126 and a light-chain variable region comprising the amino acid sequence of SEQ ID NO: 127;
(ai) an antibody or a fragment thereof comprising a heavy-chain variable region comprising the amino acid sequence of SEQ ID NO: 128 and a light-chain variable region comprising the amino acid sequence of SEQ ID NO: 129;
(aj) an antibody or a fragment thereof comprising a heavy-chain variable region comprising the amino acid sequence of SEQ ID NO: 130 and a light-chain variable region comprising the amino acid sequence of SEQ ID NO: 131;
(ak) an antibody or a fragment thereof comprising a heavy-chain variable region comprising the amino acid sequence of SEQ ID NO: 132 and a light-chain variable region comprising the amino acid sequence of SEQ ID NO: 133; and
(al) an antibody or a fragment thereof comprising a heavy-chain variable region comprising the amino acid sequence of SEQ ID NO: 300 and a light-chain variable region comprising the amino acid sequence of SEQ ID NO: 304.

9. The medicament according to any one of claims 1 to 8, wherein the antibody is a human antibody, a humanized antibody, a chimeric antibody or a single chain antibody.

10. The medicament according to any one of claims 1 to 9, wherein the cancer is a cancer expressing CAPRIN-1 protein on a cell membrane surface.

11. The medicament according to any one of claims 1 to 10, wherein the cancer is colon cancer, lung cancer, prostate cancer, ovarian cancer, pancreatic cancer, kidney cancer, breast cancer, gastric cancer, bile duct cancer, thyroid cancer, melanoma, renal cell cancer, Hodgkin's lymphoma, head and neck cancer, mesothelioma, colorectal cancer, esophageal cancer, gastroesophageal junction cancer, hepatocellular cancer, glioblastoma, urothelial cancer, urinary bladder cancer, uterus cancer, primary central nervous system lymphoma, primary testicular lymphoma, biliary tract cancer, brain tumor, leukemia, liver cancer, sarcoma, fibrosarcoma, mastocytoma, adrenal cortex cancer, Ewing's tumor, testicle cancer, basal cell cancer, lymphoma, multiple myeloma, Paget's disease or skin cancer.

12. An agent increasing drug efficacy of a pharmaceutical composition for treatment and/or prevention of cancer comprising an antibody or a fragment thereof having an immunological reactivity with CAPRIN-1 protein as an active ingredient, wherein the agent comprises a PDGFR inhibitor as an active ingredient.

13. An agent increasing drug efficacy of a pharmaceutical composition for treatment and/or prevention of cancer comprising a PDGFR inhibitor as an active ingredient, wherein the agent comprises an antibody or a fragment thereof having an immunological reactivity with CAPRIN-1 protein as an active ingredient.

14. A method for treating and/or preventing cancer, comprising administering an antibody or a fragment thereof having an immunological reactivity with CAPRIN-1 protein, and a PDGFR inhibitor together or separately to a subject.
